# EUROPEAN PATENT SPECIFICATION

(11) **EP 2 451 402 B1**
(45) Date of publication and mention of the grant of the patent: **27.04.2016**
(21) Application number: 10797417.2
(22) Date of filing: 12.07.2010
(51) Int. Cl.: A61F 2/38, A61F 2/30

(54) **KNEE JOINT DEVICE**
KNIEGELENKVORRICHTUNG
PROTHÈSE DU GENOU

(30) Priority: 10.07.2009 SE 0900971; 10.07.2009 SE 0900975; 10.07.2009 SE 0900977; 10.07.2009 SE 0900980; 30.07.2009 US 229811 P; 30.07.2009 US 229802 P; 30.07.2009 US 229815 P; 30.07.2009 US 229805 P
(43) Date of publication of application: 16.05.2012
(73) Proprietor: Kirk Promotion LTD., 114 79 Stockholm (SE)
(72) Inventor: FORSELL, Peter, CH-6300 Zug (CH)
(86) International application number: PCT/SE2010/050829
(87) International publication number: WO 2011/005210

(56) References cited:
- WO-A1-00/15153
- WO-A1-95/01139
- WO-A2-2004/037119
- US-A1- 2005 154 471
- US-A1- 2005 154 471
- US-A1- 2008 195 216
- US-A1- 2008 208 346
- US-A1- 2008 208 346
- US-B1- 6 171 340
- US-B1- 6 200 346

## Description

### FIELD OF INVENTION

The present invention relates generally to a medical device for implantation in a human patient.

### BACKGROUND

Knee replacement surgery is one of the most commons surgical procedures to date performed at more than a million patients every year worldwide. The most common reason for performing a knee replacement surgery is that the patient suffers from knee joint osteoarthritis, which is a syndrome in which a low-grade inflammation results in pain in the joints. The low-grade inflammation is caused by abnormal wearing of the cartilage that covers and acts as a cushion inside joints, which results in a decrease of the synovial fluid that lubricates the knee joint.

In conventional surgery the surgeon places a prosthesis on the lateral condyle, the medial condyle or both the medial and the lateral condyle. The prosthesis could further more comprise a contacting surface placed on the top part of the tibia bone and an artificial part replacing the knee cap.

The procedure usually takes up to two hours and the surgeon will make a single cut (10 to 30cm long) down the front of the knee. The kneecap is moved to one side to reach the knee joint. The worn or damaged surfaces are removed and the bone are shaped to fit the artificial knee joint.

The average patient age is between 65 and 75. Of these surgeries, approximately 80% are unilateral (only one knee replaced) and 20% are bilateral.

The knee joint comprises proximal contacting surfaces, being sections of the medial condyle, the lateral condyle and an area of the femoral bone between the medial and the lateral condyle and a distal contacting surface being a cross-section of the proximal part of the tibia bone. Furthermore the knee joint comprises the patella which is a, triangular bone which articulates with the femur and covers and protects the knee joint. The knee joint also comprises the minisci which are cartilaginous elements within the knee joint which serve to protect the ends of the bones from rubbing on each other. The minisci also acts as shock absorbers in the knee. There are two menisci in each knee, the medial meniscus and the lateral meniscus. Osteoarthritis is a common condition of cartilage failure that can lead to limited range of motion, bone damage and invariably, pain. Due to a combination of acute stress and chronic fatigue, osteoarthritis directly manifests itself in a wearing away of the articulating surface and, in extreme cases, bone can be exposed in the joint. Some additional examples of cartilage failure mechanisms include cellular matrix linkage rupture, chondrocyte protein synthesis inhibition, and chondrocyte apoptosis.

The closest prior art is disclosed by US 2005/0154471.

### SUMMARY

A medical device for implantation in a knee joint of a human patient is provided. The medical device having an outer surface, being curved, creating a curved outer surface along a frontal to dorsal curved length axis following the curved outer surface, having a middle section, when said medical device is implanted in a functional position in the knee joint, said middle section being placed in the middle of said curved outer surface along said length axis, said medical device further comprising a form fitted structure clasping the distal portion of the femoral bone, and at least one beyond part. The beyond part extends from the surrounding part of the curved outer surface beyond an equatorial plane of an equatorial half of the condyle, extending away from the contacting surface, on at least one condyle, on at least a part of the curved outer surface. The beyond part is adapted to have a closest distance to a centre axis of the elongated distribution of the femur, being smaller than the closest distance of a point of the maximum diameter of the femoral condyle, and if further adapted to create a more stable position of the medical device, when mounted on at least one of the femoral condyles, when implanted in a functional position in the knee joint. The medical device further comprises a through-going fixation element that is arranged in the at least one beyond part and adapted to travel from the frontal part of said medical device positioned at the frontal part of the femoral bone, through the femoral bone and into the rear part of the medical device positioned on the rear part of the femoral bone, in order to fixate the medical device clasping the distal portion of the femoral bone by the through-going fixation element, when mounted in the knee joint. Further, at least two parts of the medical device are adapted to be connected to each other after implantation in the knee joint of the human patient, wherein the two parts are mounted with a form fitted structure along a length axis of the medical device along the functional movement of the knee joint.

The medical device may comprise an inner surface, wherein said inner surface comprises: a first point, a second point, a third point, a fourth point. The inner surface, or an imaginary special extension of said inner surface, comprises a fifth point, and a sixth point. The imaginary special extension fills out a circumferential discontinuity transverse to the length axis extension. The curved inner surface along the frontal to dorsal curved length axis is hollow having an opening, wherein an imaginary circle is immersed within the opening, with maximum size, wherein any discontinuity in the circumferential extension of the end portions of the artificial femur surface, closest to the opening, along the circumferential extension of the circle, is filled out by said circle, wherein said inner surface or said imaginary circular wall created by the circle immersed within the hole, comprising the position for the fifth and sixth point, wherein all points are located on different places along an inner length axis of said inner surface, or an imaginary special extension thereof, wherein said inner length axis is adapted to be a length axis distributed in a defined relation to the outer length axis. A first straight line, reaching from said first point to said second point is parallel to a second straight line reaching from said third point to said fourth point, which in turn is parallel to a third straight line reaching from said fifth point to said sixth point, wherein: said first straight line is placed closer to the middle section of said curved outer surface than said second and third straight lines, said second straight line is longer or of equal length than said first straight line and placed between said first and third straight lines. The second straight line is longer than said third straight line, when said medical device is placed in the functional position in the knee joint, such that the medical device is adapted to clasp the distal portion of the femoral bone.

According to one embodiment, the medical device comprises a first articulating surface adapted replaced the surface of the contacting surface of the medial condyle, a second articulating surface adapted to replace the contacting surface of the lateral condyle and a third articulating surface adapted to replace the contacting surface of the patella.

According to another embodiment, the through-going fixation element is attached to the medical device using a fixed arrangement at one portion and an adjustable arrangement at the other portion of the medical device.

According to one embodiment, the through-going fixation element comprises a threaded portion, and the adjustable arrangement comprises a portion with corresponding threads.

According to one embodiment, the through-going fixation element is assisted by an adhesive.

According to one embodiment, the medical device could be adapted to be placed on an intact distal portion of the femoral bone.

The knee joint includes a contacting surface of the distal portion of the femoral bone or femur, and a tibia surface, including a contacting surface of the proximal portion of the tibia bone or tibia. The contacting surfaces at least partly contacting each other in a functional knee joint, carrying weight in the knee joint, the femur also contacts the patella in the patella femur contacting surface contacting the patella contacting surface. The femoral bone has an elongated distribution leading distally to a medial and lateral condyle of the femur, wherein a center axis of the elongated distribution of the femoral bone being the elongated femur center axis, wherein the condyles are integrated in the femur in an upside-down Y-configuration having one medial and one lateral condyle with a rounded form, towards a ball shaped condyle, integrated in the upside down Y-shaped double neck of the femur. The condyle has an equatorial plane dividing the condyle in half of an imaginary complete ball shaped configuration, when imagining the condyle having a complete towards a ball shape configuration also through the neck of the femur. The condyles has a center axis substantially in the centre of both the medial and lateral condyle, perpendicular to the elongated femur centre axis, defined as the femur condyles center axis, placed dorsally to the elongated femur center axis. The condyles has a part of the circumference being the femur contacting surface, extending in frontal-dorsal direction following the movement of the knee, less than 180 degrees. The equatorial plane has a center axis extending perpendicularly from the center thereof, defining the condyle equatorial plane center axis. The hollow medical device comprises a central part and a surrounding part, the central part has a center axis in the center thereof, being aligned with the condyle equatorial plane center axis, wherein the surrounding part surrounding the surface of the femur condyle or a partly modified femur condyle surface not including the central part. The artificial knee joint surface, comprising an artificial femur surface adapted to at least partly replace and replacing at least one of; the femur contacting surface of the medial condyle and the femur contacting surface of the lateral femoral condyle and the patella femur contacting surface, and adapted to be placed to contact, at least one of and at least a part of, the tibia contacting surface or an artificial replacement therefore and the patella contacting surface or an artificial replacement therefore, when mounted in the knee joint. The artificial femur surface is hollow and distally has at least to a part an outer surface being curved, the artificial femur surface comprising; at least one first beyond part extending from the surrounding part of the artificial femur surface beyond the equatorial plane of the equatorial half of the condyle, extending away from the contacting surface, on at least one of the medial and lateral condyle, on at least a part of the artificial femur surface. The at least one beyond part is extending at least to a part on both a circumferential quarter of the equatorial plane placed; a) most dorsal and b) most frontal of the artificial femur surface, when the artificial femur surface is mounted on the femur in its functional position in the knee joint. On the inner surface of the hollow artificial femur surface, the at least one first beyond part is adapted to have, a closest distance to the condyle equatorial plane center axis, being smaller than the largest distance from the inner surface of the artificial femur surface to the condyle equatorial plane center axis, the closest and longest distance are extending from both of the two circumferential quarters of the equatorial plane placed; a) most dorsal and b) most frontal of the artificial femur surface, thus adapted to create and creating a more stable position of said artificial femur surface, when mounted on said at least one of the femoral condyles in the functional position in the knee joint.

The knee joint includes a contacting surface of the distal portion of the femoral bone or femur, and a tibia surface, including a contacting surface of the proximal portion of the tibia bone or tibia. The contacting surfaces at least partly contacting each other in a functional knee joint, carrying weight in the knee joint, the femur also contacts the patella in the patella femur contacting surface contacting the patella contacting surface. The femoral bone has an elongated distribution leading distally to a medial and lateral condyle of the femur a center axis of the elongated distribution of the femoral bone is the elongated femur center axis, the condyles are integrated in the femur in an upside-down Y-configuration having one medial and one lateral condyle with a rounded form, towards a ball shaped condyle, integrated in the upside down Y-shaped double neck of the femur. The condyle has an equatorial plane dividing the condyle in half of an imaginary complete ball shaped configuration, when imagining the condyle having a complete towards a ball shape configuration also through the neck of the femur. The condyles has a center axis substantially in the centre of both the medial and lateral condyle, perpendicular to the elongated femur centre axis, defined as the femur condyles center axis, placed dorsally to the elongated femur center axis. The condyles has a part of the circumference being the femur contacting surface, extending in frontal-dorsal direction following the movement of the knee, less than 180 degrees. A line from the femur contacting surface's most proximal-dorsal point in the periphery of the condyle, substantially in the middle of the contacting surface in medial-lateral direction, is extending perpendicular through the femur condyle center axis until it reaches the periphery of the frontal-distal part of the condyle. Such line extended to a plane, horizontally in medial-lateral direction, defines a special equatorial plane, including the complete femur contacting surface in the equatorial divided half of an imaginary complete ball shaped configuration of the condyle. The special equatorial plane has a center axis extending perpendicularly from the center thereof, defining the condyle special equatorial plane center axis. The medical device, comprises a central part and a surrounding part, the central part is aligned with a medical device center axis and the surrounding part surrounding the surface of the femur condyle or a partly modified femur condyle surface not including the central part. The artificial knee joint surface, comprises an artificial femur surface adapted to at least partly replace and replacing at least one of; the femur contacting surface of the medial condyle and the femur contacting surface of the lateral femoral condyle and the patella femur contacting surface, and adapted to be placed to contact, at least one of and at least a part of, the tibia contacting surface or an artificial replacement therefore and the patella contacting surface or an artificial replacement therefore, when mounted in the knee joint. The artificial femur surface is hollow and distally has at least to a part an outer surface being curved. The artificial femur surface comprising at least one first beyond part extending from the surrounding part of the artificial femur surface beyond the special equatorial plane of the equatorial half of the condyle including the complete contacting surface, extending away from the contacting surface, on at least one of the medial and lateral condyle, on at least a part of the artificial femur surface. The at least one beyond part is extending at least to a part on both a circumferential quarter of the equatorial plane placed; a) most dorsal and b) most frontal of the artificial femur surface, when said artificial femur surface is mounted on the femur in its functional position in the knee joint. On the inner surface of the hollow artificial femur surface, the at least one first beyond part is adapted to have, a closest distance to the condyle special equatorial plane center axis, being smaller than the largest distance from the inner surface of the artificial femur surface to the condyle special equatorial plane center axis, the closest and longest distance are extending from both of the two circumferential quarters of the equatorial plane placed; a) most dorsal and b) most frontal of the artificial femur surface, thus adapted to create and creating a more stable position of said artificial femur surface, when mounted on said at least one of the femoral condyles in the functional position in the knee joint.

The knee joint comprises a femur surface, being a contacting surface of the distal portion of the femoral bone or femur, and a tibia surface, being a contacting surface of the proximal portion of the tibia bone or tibia. The contacting surfaces are at least partly contacting each other in a functional knee joint. The femoral bone have an elongated distribution leading distally to a left and right condyle of the femur, the condyles having a cross-section parallel to the elongated distribution with a substantially round shaped configuration distally on the condyles, when viewed from the lateral or medial side of said femoral bone. The section have a maximum diameter, substantially perpendicular to the elongated distribution of the femoral bone, wherein the medical device comprises an artificial knee joint surface comprising; an artificial femur surface adapted to at least partly replace and replacing the joint surface of at least one of the femoral condyles, placed distally on said femoral bone and adapted to be placed to contact, at least partly, with said tibia contacting surface, or an artificial replacement therefore, when mounted in the joint. The artificial femur surface, comprising at least one first beyond part adapted to cover and/or go into the bone of the femur on at least a part of the femur beyond the maximum diameter of the condyle in the proximal direction of the elongated distribution of the femur, when mounted on the femur in its functional position in the joint. The at least one first beyond part is adapted to have a closest distance to a centre axis of the elongated distribution of the femur, being smaller than the closest distance of a point of said maximum diameter of the femoral condyle and the centre axis of the elongated distribution of the femur. Thereby the medical device is adapted to create and creating a more stable position of the
artificial femur surface, when mounted on at least one of the femoral condyles in the functional position.

According to one embodiment, the medical device has a substantially horseshoe-shaped section having a base, two ends, an inner surface and an outer surface. A first straight line reaches from a first end of said inner surface of said horseshoe-shaped section to a second end of said horseshoe-shaped section; the first straight line is positioned in an insertion opening of said medical device. A second straight line, reaches between two points on said inner surface of said horseshoe-shaped section. The first straight line and the second straight line are parallel, the first straight line is shorter than the second straight line, and the second straight line is placed closer to the base of the horseshoe-shaped section than the first straight line.

According to one embodiment the medical device is adapted to be fixated to the medial condyle of a human patient, which could be done through the medical device being adapted to clasp said medial condyle. However it is equally conceivable that the medical device is adapted to be fixated to the lateral condyle of a human patient, which could be done through the medical device being adapted to clasp said lateral condyle.

According to another embodiment the medical device is adapted to be fixated to both the medial condyle and the lateral condyle of the human patient, which could be done through the medical device being adapted to clasp both said medial condyle and said lateral condyle.

According to one embodiment the medical device further have a largest cross sectional distance and at least one movable member for varying said largest cross sectional distance of the medical device. The moveable member could comprise at least one elastic member, which could be at least one part of the medical device adapted to enable mounting of the medical device on at least one of the lateral condyle and the medial condyle of the human patient.

The medical device according to any of the embodiments herein could be adapted to have a variable closest distance between said beyond part and said center axis allowing mounting of said artificial femur surface onto at least one of the femur condyles having a first larger closest distance, having a second smaller closest distance when implanted in the functional position in the knee joint.

According to one embodiment the medical device is adapted to have an opening with a variable size, larger during mounting and smaller, when implanted in the functional position in the knee joint.

### IMPLANTATION

The knee joint comprises a first contacting surface, being a surface of the distal portion of the femoral bone, and a second contacting surface, being a surface of the proximal portion of the tibia bone. According to one embodiment the medical device is adapted to be in connection with the first contacting surface when implanted in the knee joint. The connection could be a direct connection with the first contacting surface, or an indirect connection with the first contacting surface. In the embodiments where the connection is an indirect connection a material could be positioned between the medical device and the first contacting surface. The material could be a material selected from a group consisting of: adhesive materials, elastic materials and bone cement.

The knee joint comprises a first contacting surface, being a surface of the distal portion of the femoral bone, and a second contacting surface, being a surface of the proximal portion of the tibia bone. According to one embodiment the medical device comprises three parts wherein at least two parts are adapted to be mechanically connected to each other when implanted in the knee joint, the at least two parts are fixated to the first contacting surface. A third part is fixated to the second contacting surface. The at least two parts fixated to the first contacting surface are in moveable connection with the third part fixated to the second contacting surface, when the medical device is implanted in the knee joint. The movable connection could be a movable direct connection or a movable indirect connection.

According to one embodiment a material is positioned between at least two parts fixated to said first contacting surface and said third part fixated to said second contacting surface. The material could be a material adapted to reduce friction in the knee joint. Such as a lubricating fluid, which preferably is a biocompatible lubricating fluid such as hyaluronic acid. According to another embodiment the material adapted to reduce friction is a fluorpolymer material, such as PTFE.

### ATTACHMENT

According to one embodiment the medical device is adapted to be fixated to a bone of the human patient using a fixation element selected from a group consisting of: at least one screw, at least one pin, at least one portion of at least one of the parts adapted to be introduced into the other part, the parts being adapted to be sliding into the other part, form fitting, welding, adhesive, pin, wire, a ball mounted into a cup being portions of said parts, a male portion of one part mounted into a female portion of the other part, a key introduced into a lock being portions of said parts, band, or other mechanical connecting members.

According to one embodiment the medical device is adapted to be fixated to the distal part of the femoral bone using a through-going fixation element, which could be a through-going fixation element adapted to travel from the frontal part of the medical device, positioned at the frontal part of the femoral bone, through the femoral bone and into the rear part of the medical device, positioned on the rear part of the femoral bone. The through-going fixation element could be a through-going fixation element selected from a group consisting of: screws, pins, wire, rivets, band and cord. The through-going fixation element could further be assisted by an adhesive.

The at least two parts adapted to be connected to each other after implantation in said knee joint, according to any of the embodiments, could be connected to each other using at least on element selected from a group consisting of: at least one screw, at least one pin, at least one portion of at least one of the parts adapted to be introduced into the other part, the parts being adapted to be sliding into the other part, form fitting, welding, adhesive, wire, a ball mounted into a cup being portions of said parts, a male portion of one part mounted into a female portion of the other part, a key introduced into a lock being portions of said parts, band, or other mechanical connecting members.

According to one embodiment the medical device is adapted to be fixated to the distal part of the femoral bone without penetration of the cortex of said femoral bone.

The distal part of the femoral bone has a largest cross sectional distance. According to one embodiment the medical device comprises an opening smaller than the largest cross sectional distance when the medical device is mounted on the distal part of the femoral bone in a functional position. The opening could be larger than the largest cross sectional distance when the medical device travels over the distal part of the femoral bone.

The medical device according to any of the embodiments could be made of a single material, or at least a part adapted to clasp the medial and/or the lateral condyle or the proximal part of the tibia bone, could be made from the same material.

### MATERIAL

The elastic member, according to any of the embodiments above, could comprise an elastic material, which could be elastic polymer material or an elastic metal material. In embodiments where the elastic material is an elastic polymer material the elastic polymer material could be an elastic polymer material selected from a group consisting of: polystyrene, poly(ethylene-butylene), polyurethane elastomeric material, polyamide elastomeric materials, polyester elastomeric materials, elastic copolymers of ethylene, vinyl acetates, unsaturated aliphatic monocarboxylic acids and monocarboxylic acids.

In embodiment where the elastic material is an elastic metal material, the elastic material could be a biocompatible metal material, such as titanium or tantalum. It is also conceivable that the material is a multi-layered material in which at least one layer is adapted to protect against the body cells. According to one embodiment at least one layer is a Parylene layer.

The medical device according to any of the embodiments herein could have a variable closest distance being variable due to a movable beyond part, the beyond part could comprise a slit for being movable. According to another embodiment the beyond part is bendable.

The movable beyond part could comprise a locking position to lock the beyond part in its position, when implanted in the functional position in the knee joint.

The medical device could comprising a locking member for locking the movable beyond part in a locking position, when implanted in the functional position in the knee joint. The locking member could comprising at least one of a locking wire and a locking ring.

According to one embodiment the size of the opening is adapted to be fixed in a locking position, when implanted in the functional position in the knee joint.

The medical device according to anyone of the embodiments could comprise a locking wire to lock the opening to be fixed in a locking position, when implanted in the functional position in the knee joint.

The medical device according to any of the embodiments could comprise a locking ring for locking the opening to be fixed in a locking position, when implanted in the functional position in the knee joint.

The locking wire could be adapted to pass through the femur condyle, when implanted in the functional position in the knee joint, or the locking ring could be adapted to be mounted onto the femur condyle, when implanted in the functional position in the knee joint.

According to yet another embodiment the medical device could comprise an artificial femur surface, comprising at least one of an artificial medial and lateral femur surface, adapted to contact the tibia surface or an artificial replacement therefore.

In another embodiment the medical device has said beyond part in it's beyond extension from the surrounding part of the artificial femur surface, beyond the special equatorial plane of the equatorial half of the condyle, when implanted in the knee joint, is adapted to have one or more beyond parts related to the circumferential extension of the artificial femoral surface, the beyond parts is furthermore adapted to extend in different or similar lengths, in all cases adapted to be mounted around the femur condyle beyond the equatorial half of the condyle in such a way that the beyond parts fixates the artificial femur surface onto the at least one femur condyle.

Said beyond parts may comprise at least one first beyond part plus one or more further beyond part, wherein if a curved line is interconnecting said beyond parts circumferentially following the artificial femur surface, without connecting back to the first beyond part again, from the beginning of the first beyond part to the end of the last beyond part, the totsl circumferential extension is more than 180°.

The hollow medical device may have the opening adapted to be fitted with the femur condyle, at least partly in proximal direction, wherein the center axis of the central part of the medical device, extends out from the hollow medical device in the opening and is adapted to be centered in said opening, at least in dorsal to frontal direction.

The center axis may be adapted to be centered perpendicularly to said center axis in said smallest opening, along said center axis, at least in dorsal to frontal direction.

### METHOD

The medical device may be implanted by: cutting the skin of a human patient, dissecting an area of the knee joint, introducing the medical device according to claim 1 into the knee joint, attaching the medical device such that said medical device clasps a portion of a bone of the knee joint.

The medical device may further be implanted in a knee joint by: inserting at least one needle or a tube like instrument into the patient's knee joint, using the needle or tube like instrument to fill the joint with a fluid, placing at least two arthroscopic trocars in the joint or inserting the camera and at least one dissecting tool direct into the knee joint, inserting said camera through one of the arthroscopic trocars or direct into the knee joint, inserting said at least one dissecting tool through one of the at least two trocars or direct into the knee joint, introducing the medical device according to claim 1 into the knee joint, attaching the medical device such that said medical device clasps a portion of a bone of the knee joint.

The medical device may be fixated to a portion of a bone of the knee joint using a fixation element selected from a group consisting of: at least one screw, at least one pin, at least one portion of at least one of the parts adapted to be introduced into the other part, the parts being adapted to be sliding into the other part, form fitting, welding, adhesive, pin, wire, a ball mounted into a bowl being portions of said parts, a male portion of one part mounted into a female portion of the other part, a key introduced into a lock being portions of said parts, band, and other mechanical fixation elements.

Further, the medical device may be fixated to a portion of a bone of the knee joint using a through-going fixation element.

The medical device could further be fixated to a portion of a bone of the knee joint using a through-going fixation element and fixating said through-going fixation element from the frontal part of said medical device positioned at the frontal part of the femoral bone, through the femoral bone and into the rear part of the medical device positioned on the rear part of the femoral bone.

An adhesive may be applied for assisting the through-going fixation element in the fixation of the medical device.

The medical device may be fixated to a portion of a bone of the knee joint using a fixation element selected from a group consisting of: at least one screw, at least one pin, at least one portion of at least one of the parts adapted to be introduced into the other part, the parts being adapted to be sliding into the other part, form fitting, welding, adhesive, pin, wire, a ball mounted into a bowl being portions of said parts, a male portion of one part mounted into a female portion of the other part, a key introduced into a lock being portions of said parts, band, and other mechanical fixation elements.

The medical device may further be fixated to a portion of a bone of the knee joint using a through-going fixation element. The medical device may be fixated to a portion of a bone of the knee joint using a through-going fixation element and fixating the through-going fixation element from the frontal part of said medical device positioned at the frontal part of the femoral bone, through the femoral bone and into the rear part of the medical device positioned on the rear part of the femoral bone.

Further, an adhesive could be applied for assisting the said through-going fixation element in the fixation of said medical device.

According to yet another embodiment the medical device comprises at least two medical device parts, wherein the medical device may be introduced by: introducing a first part of the medical device, introducing a second part of the medical device, and connecting said first part of the medical device to said second part of the medical device.

The medical device could comprise at least two medical device parts, wherein the medical device may be introduced by: introducing a first part of the medical device, introducing a second part of the medical device, and connecting said first part of the medical device to said second part of the medical device.

Please note that any embodiment or part of embodiment, described herein may be combined in any way.

### BRIEF DESCRIPTION OF DRAWINGS

The invention is now described, by way of example, with reference to the accompanying drawings, in which:
Fig. 1 shows a frontal view of the right leg of a human patient when an incision in a surgical method has been performed,
Fig. 2 shows a frontal view of the right leg of a human patient when the knee joint is being prepared,
Fig. 3 shows the knee joint of a human patient,
Fig. 4a shows a medical device according to an exemplifying embodiment describing the technical area of the invention, comprising several medical device parts,
Fig. 4b shows a medical device according to an exemplifying embodiment describing the technical area of the invention, comprising several medical device parts when assembled,
Fig. 5 shows the lower part of the femoral bone when a medical device according to an exemplifying embodiment describing the technical area of the invention, comprising several medical device parts, are being fixated,
Fig. 6 shows the lower part of the femoral bone when a medical device according to an exemplifying embodiment describing the technical area of the invention, comprising several medical device parts, have been fixated,
Fig. 7a shows a medical device according to an exemplifying embodiment describing the technical area of the invention, which comprises several medical device parts adapted for fixation to the upper part of the tibia bone,
Fig. 7b shows a medical device according to an exemplifying embodiment describing the technical area of the invention, which comprises several medical device parts adapted for fixation to the upper part of the tibia bone when assembled,
Fig. 8 shows a medical device according to an exemplifying embodiment describing the technical area of the invention, comprising several medical device parts, when said medical device is being fixated to the tibia bone,
Fig. 9 shows the medical device according to an exemplifying embodiment describing the technical area of the invention, comprising several medical device parts, when said medical device has been fixated to the tibia bone,
Fig. 10a shows a medical device according to an exemplifying embodiment describing the technical area of the invention, when the medical device is operable, in perspective,
Fig. 10b shows a medical device according to an exemplifying embodiment describing the technical area of the invention, when the medical device is operable, from above,
Fig. 10c shows a medical device according to an exemplifying embodiment describing the technical area of the invention, when the medical device is operable, in its folded state,
Fig. 10d shows a medical device according to an exemplifying embodiment describing the technical area of the invention, when the medical device is operable, in further detail,
Fig. 11 shows the medical device according to an exemplifying embodiment describing the technical area of the invention, comprising several medical device parts being fixated to each other by means of self locking elements,
Fig. 12 shows the medical device according to an exemplifying embodiment describing the technical area of the invention, comprising several medical device parts being fixated to each other by means of form fitting,
Fig. 13a shows a medical device according to an exemplifying embodiment describing the technical area of the invention, comprising several ring shaped medical device parts,
Fig. 13b shows a medical device according to an exemplifying embodiment describing the technical area of the invention, comprising several ring shaped medical device parts, when assembled,
Fig. 13c shows a medical device according to an exemplifying embodiment describing the technical area of the invention, comprising several ring shaped medical device parts, in further detail,
Fig. 14 shows the left leg of a human patient in section when a medical device has been implanted,
Fig. 15 shows the medical device according to an embodiment where the medical device is adapted to clasp a bone of the knee joint.
Fig. 16 shows the medical device according to an embodiment where the medical device comprises two parts adapted to be interconnected by means of pins and holes,
Fig. 17a shows the medical device according to an embodiment where the medical device comprises two parts adapted to be interconnected by means of a locking member and a keyhole,
Fig. 17b shows the locking member and keyhole in further detail,
Fig. 17c shows the medical device when it is being assembled in a first state,
Fig. 17d shows the medical device when it is being assembled in a second state,
Fig. 17e shows the locking member and keyhole in further detail,
Fig. 18a shows the medical device according to an embodiment where the medical device comprises two parts adapted to be interconnected by means of a locking member and a keyhole,
Fig. 18b shows the locking member and keyhole in further detail,
Fig. 19a shows the medical device according to an embodiment where the medical device comprises two parts adapted to be interconnected by means of locking members and holes,
Fig. 19b shows the medical device according to an embodiment where the medical device comprises two parts adapted to be interconnected by means of locking members and holes,
when being assembled,
Fig. 19c shows the medical device according to an embodiment where the medical device comprises two parts adapted to be interconnected by means of locking members and holes,
when assembled,
Fig. 19d shows the locking members and hole in section,
Fig. 20a shows the medical device comprising an even surface in perspective,
Fig. 20b shows a cross-section of the medical device comprising an even surface,
Fig. 21a shows the left leg of a human patient when a medical device comprising two layers have been implanted,
Fig. 21b shows the weight carrying surface comprising two carrying surface parts,
Fig. 21c shows the flexible layer of the medical device,
Fig. 22 shows the medical device according to one embodiment, when implanted in the knee joint of a human patient,
Fig. 23 shows the medical device according to an embodiment when the medical device is adapted to clasp the medial and lateral condyle.
Fig. 24 shows the medical device according to an embodiment wherein the medical device comprises five medical device parts,
Fig. 25 shows a second embodiment wherein the medical device comprises five medical device parts,
Fig. 26 shows the medical device when being positioned on the distal part of the femoral bone,
Fig. 27 shows a flowchart of a surgical method,
Fig. 28 shows a flowchart of an arthroscopic method,
Fig. 29 shows a medical device according to an exemplifying embodiment describing the technical area of the invention, wherein the medical device comprises four moveable sections,
Fig. 30a shows a medical device according to an exemplifying embodiment describing the technical area of the invention, wherein the medical device comprises four moveable sections,
in a first state,
Fig. 30b shows a medical device according to an exemplifying embodiment describing the technical area of the invention, wherein the medical device comprises four moveable sections,
in a second state,
Fig. 31 shows the medical device according to an exemplifying embodiment describing the technical area of the invention, wherein the medical device comprises four moveable sections,
when inserted into a knee joint in a surgical method,
Fig. 32 shows a medical device according to an exemplifying embodiment describing the technical area of the invention, wherein the medical device comprises four moveable sections,
when inserted into a knee joint in an arthroscopic method,
Fig. 33 shows a medical device according to an exemplifying embodiment describing the technical area of the invention, wherein the medical device comprises four slits,
Fig. 34 shows a band adapted to fixate the medical device according to fig. 33.
Fig. 35 shows the medical device according to fig. 33 when fixated to the medial condyle of the femoral bone,
Fig. 36 shows the right leg of a human patient when a surgical procedure is being performed,
Fig. 37 shows the right leg of a human patient when an arthroscopic procedure is being performed,
Fig. 38 shows the knee joint of a human patient when an implantable lubrication system has been implanted,
Fig. 39 shows a frontal view of a human patient when an implantable lubrication system has been implanted.

### DETAILED DESCRIPTION

A length axis of the femoral bone is to be understood as an axis which extends in the direction of the length of the femoral bone from the proximal part of the femoral bone to the distal part of the femoral bone.

An axis of the lateral condyle and the medial condyle is to be understood as an axis which is perpendicular to a length axis of the femoral bone. The functional knee movements of a natural knee joint are performed in around an axis of the lateral and medial condyle.

Biocompatible material is to be understood as being a material with low level of immune response. Biocompatible materials are sometimes also referred to as biomaterials. Analogous is biocompatible metals a biocompatible metal with low immune response such as titanium or tantalum. The biocompatible metal could also be a biocompatible alloy comprising at least one biocompatible metal.

A metal alloy is to be understood as a mixture of two or more elements in solid solution in which the major component is a metal. A steel alloy is hence an alloy wherein one of the components is steel which in turn is an alloy of iron and carbon. A titanium alloy is hence an alloy wherein one of the components is titanium.

Elasticity is to be understood as a materials ability to deform in an elastic way.

Carrying surface and weight carrying surface is to be understood as a surface adapted to carry weight inside of said knee joint.

Form fitting is to be understood as an element having a part or section which is adapted to enable a mechanical connection of said element to at least one other element using said part or section. Form fitted structure is a structure of an element which enables form fitting. One type of form fitting clasps an element such as a human bone, the fixation is then created through said clasping.

Functional knee movements are to be understood as movements of the knee that at least partly correspond to the natural movements of the knee. On some occasions the natural movements of the knee joint might be somewhat limited or altered after knee joint surgery, which makes the functional knee movements of a knee joint with artificial surfaces somewhat different than the functional knee movements of a natural knee joint.

The functional position of an implantable medical device or prosthesis is the position in which the knee joint can perform functional knee movements.

Functional knee joint is a knee joint that can perform functional knee movements either with or without an implanted medical device or prosthesis.

Full functional size is to be understood as the size of the medical device when said medical device is implanted in the knee joint.

The medical device according to any of the embodiments could comprise at least one material selected from a group consisting of: polytetrafluoroethylene (PTFE), perfluoroalkoxy (PFA) and fluorinated ethylene propylene (FEP). It is furthermore conceivable that the material comprises a metal alloy, such as cobalt-chromium-molybdenum or titanium or stainless steel, or polyethylene, such as cross-linked polyethylene or gas sterilized polyethylene. The use of ceramic material is also conceivable, in the contacting surfaces or the entire medical device such as zirconium or zirconium dioxide ceramics or alumina ceramics. The part of the medical device in contact with human bone for fixation of the medical device to human bone could comprise a poorhouse structure which could be a porous micro or nano-structure adapted to promote the growth-in of human bone in the medical device for fixating the medical device. The porous structure could be achieved by applying a hydroxy-apatite (HA) coating, or a rough open-pored titanium coating, which could be produced by air plasma spraying, a combination comprising a rough open-pored titanium coating and a HA top layer is also conceivable. The contacting parts could be made of a self lubricated material such as a waxy polymer, such as PTFE, PFA, FEP, PE and UHMWPE, or a powder metallurgy material which could be infused with a lubricant, which preferably is a biocompatible lubricant such as a Hyaluronic acid derivate. It is also conceivable that the material of contacting parts or surfaces of the medical device herein is adapted to be constantly or intermittently lubricated. According to some embodiments the parts or portions of the medical device could comprise a combination of metal materials and/or carbon fibers and/or boron, a combination of metal and plastic materials, a combination of metal and carbon based material, a combination of carbon and plastic based material, a combination of flexible and stiff materials, a combination of elastic and less elastic materials, Corian or acrylic polymers.

In the following a detailed description of embodiments will be given. In the drawing figures, like reference numerals designate identical or corresponding elements throughout the several figures. It will be appreciated that these figures are for illustration only and are not in any way restricting the scope of the invention. Thus, any references to direction, such as "up" or "down", are only referring to the directions shown in the figures. Also, any dimensions etc. shown in the figures are for illustration purposes.

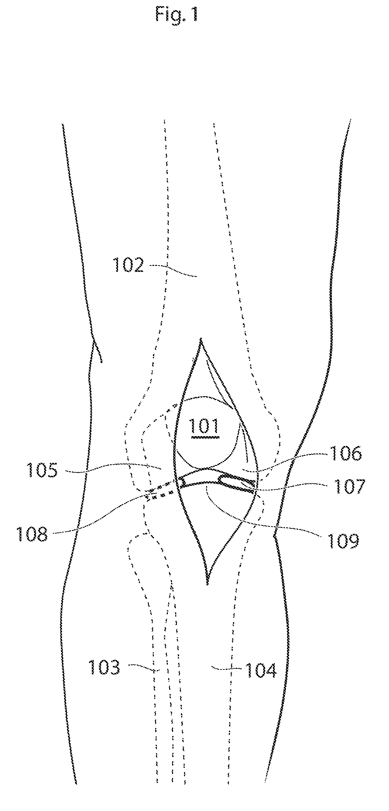

Fig. 1 shows the right leg of a human patient. The femoral bone 102 having a distal part comprising the lateral condyle 105, the medial condyle 106 and an area between said lateral and said medial condyle 131. The sections of the distal part of the femoral bone 102 comprises contacting surfaces of the knee joint. The knee joint furthermore comprises the patella 101, which is a triangular bone which articulates with the femur 102 and covers and protects the knee joint. The knee joint also comprises the minisci 107, 108 which are cartilaginous elements within the knee joint which serve as articulating surfaces to protect the ends of the bones from rubbing on each other. The minisci 107, 108 also acts as shock absorbers in the knee joint, to absorb the shocks from the movement of the human patient. There are two menisci 107,108 in each knee, the medial meniscus 107 and the lateral meniscus 108. In patients with osteoarthritis the menisci 107, 108 which acts as articulating surfaces i.e. weight carrying surfaces are worn away and, in extreme cases, bone can be exposed in the joint. The knee joint is protected by the knee joint capsule 132 also known as the articular capsule of the knee joint or the capsular ligament of the knee joint. The knee joint capsule 132 is wide and lax; thin in front and at the side; and contains the patella 101, ligaments, menisci 107,108, and bursae, which are small fluid-filled sacs made of white fibrous tissue. The knee joint capsule 132 consists of a synovial and a fibrous membrane separated by fatty deposits anteriorly and posteriorly.

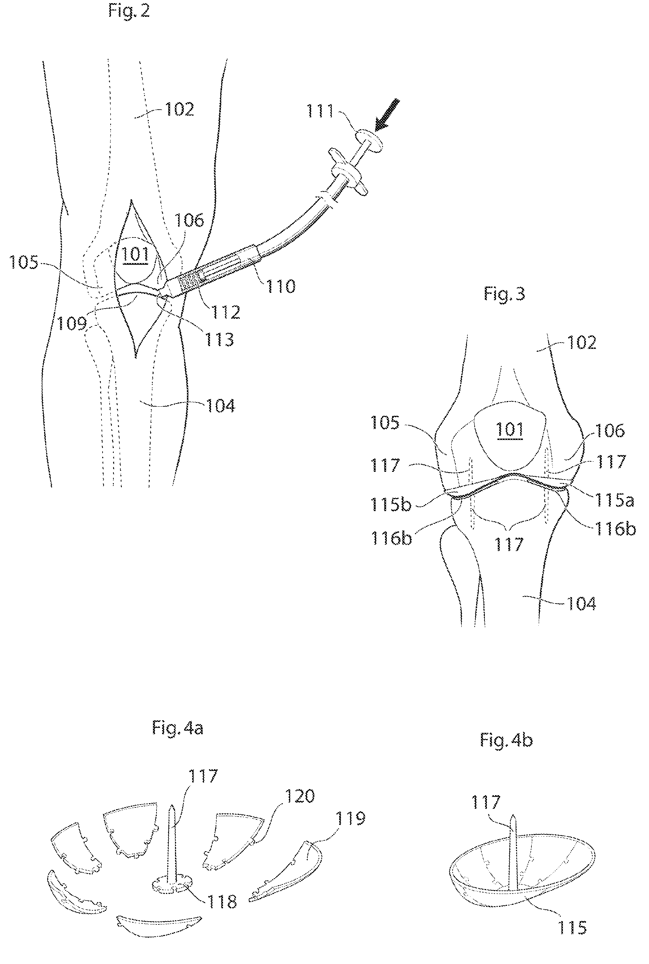

Fig. 2 shows the right leg in an embodiment where the distal surface of the femoral bone 102 and the proximal portion of the tibia bone 104 is prepared for the insertion of a medical device. The preparation of the surfaces includes the injection of an adhesive 112 on to the contacting surfaces. The adhesive 112 is preferably injected using an injecting member 110 which includes a injecting force creation member 111 which could be powered manually or by means of an operating device, such as an electrical, hydraulic or pneumatic motor.

Fig. 3 shows the knee joint with the knee joint capsule 132 removed. A medical device 115a has been placed on the medial condyle 106. The medical device is fixated using a fixation member 117 which is inserted into the distal part of the femoral bone 102. Furthermore a medical device 116a comprising a contacting surface i.e. weight carrying surface has been placed on the proximal portion of the tibia bone 104 using fixation members 117 inserted into the tibia bone 104. An equivalent pare of medical devices 115b, 116b has been fixated to the lateral condyle 105 using fixation members 117. The fixation members 117 could be screws or pins which could be fixated by means of mechanical fixation which could be assisted or replaced by adhesive.

Fig. 4a shows an exemplifying embodiment describing the technical area of the invention, wherein a medical device comprises multiple medical device parts 119 which are adapted to be connected to each other to form a functional knee joint surface. The multiple medical device parts 119 can be fixated to a base part 118 which could comprise the fixation member 117. According to the embodiment shown in fig. 4a, said multiple parts are adapted to be connected to each other by means of form fittings 120, i.e. the multiple parts 119 comprises mechanical elements adapted to lock to each other.

Fig 4b shows the medical device according to the embodiment described with reference to fig. 4a, in which the medical device comprises multiple medical device parts 119, when said medical device has been assembled and fixated by means of form fitting. According to one embodiment said form fitting can be assisted with adhesive. Fig. 4b further shows an example of the medical device comprising a central part 1601 and a surrounding part 1602, in this case the central part comprises the fixation member 117.

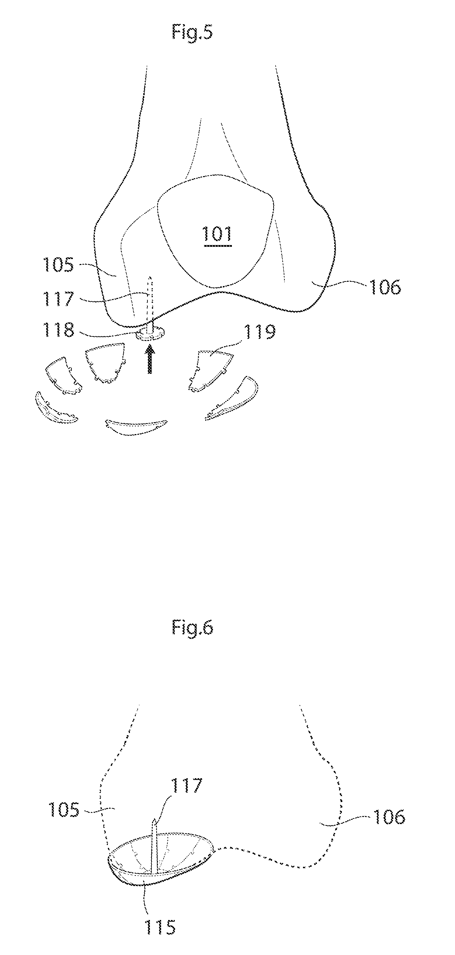

Fig. 5 shows an exemplifying embodiment describing the technical area of the invention, wherein a medical device comprises multiple medical device parts 119, when said medical device is being fixated to the lateral condyle 105 of the femoral bone 102 of a human patient. The fixation member 117 is fixated to the cortical and cancellous bone of the femoral bone 102 and attached to a base part 118. The artificial knee joint surface parts 119 are then attached to the base part 118 by means of form fitting. According to another embodiment (not shown) each of the knee joint surface parts 119 are adapted to be individually fixated to the femoral bone by means of a fixating member 117, in which case the individual knee joint surface parts 119 can be attached to each other by means of said individual fixation.

Fig. 6 shows an exemplifying embodiment describing the technical area of the invention, wherein a medical device comprises multiple medical device parts 119, when said medical device is fixated to the lateral condyle 105 of said human patient. Fig 6 further shows the condyles 105,106 having an imaginary complete ball shape 1701 which abuts the periphery of the condyles 105,106.

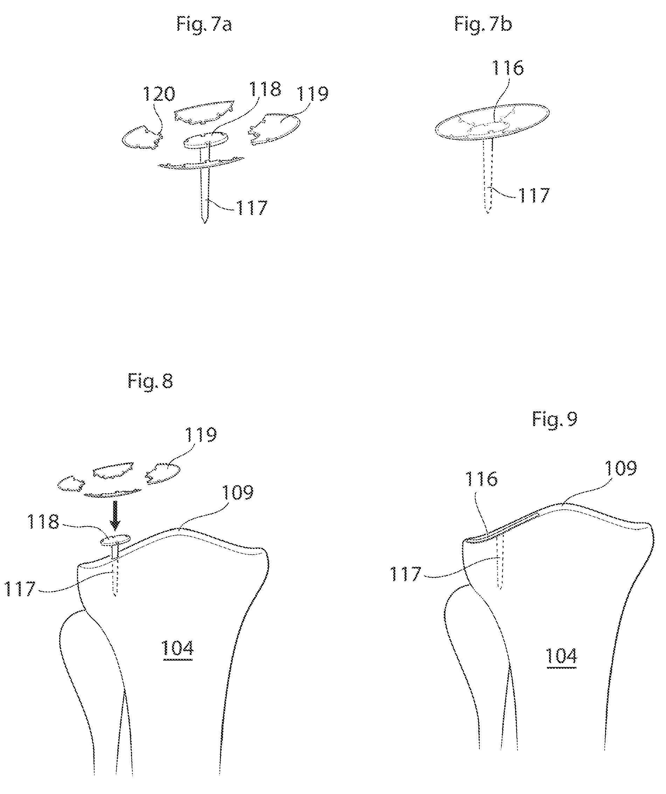

Fig. 7a shows a medical device according to an exemplifying embodiment describing the technical area of the invention, wherein a medical device is adapted to be fixated to the tibia bone of proximal part of the tibia bone of the human patient. The medical device comprises multiple artificial surface parts 119 which are adapted to be fixated to each other after said medical device has been implanted in the knee joint of a human patient. According to the embodiment shown in fig. 7a the medical device comprises a base part 118 which in turn comprises a fixation member which is adapted to mechanically fixate the medical device to the tibia bone 104 of a human patient.

Fig. 7b shows the medical device according to an exemplifying embodiment describing the technical area of the invention, wherein a medical device is assembled to form an artificial knee joint surface 116 adapted to be fixated to the tibia bone of the human patient by means of the fixating member 117. According to other embodiment the fixating member 117 is assisted or replaced by an adhesive.

Fig. 8 shows the medical device according to the embodiment of figs. 7a and 7b, when said medical device is being fixated to the lateral part of contacting surface 109 of the tibia bone 104 by means of a fixation member 117 adapted to be mechanically fixated to the cortical and cancellous bone of the tibia bone 104. Preferably the fixating member 117 is a screw or pin adapted to fixated only by means of its mechanical shape or assisted with a chemical agent such as an adhesive.

Fig. 9 shows an exemplifying embodiment describing the technical area of the invention, wherein a medical device comprises ing an artificial knee joint surface 116 adapted to be fixated to the contacting surface 109 of the tibia bone 104 on the lateral side thereof. The medical device is now assembled and fixated to the tibia bone by means of a fixating member 117 in the tibia bone 104. It is however equally conceivable that the medical device is fixated to the tibia bone on the medial side thereof, or to the tibia bone on both the lateral and the medial side thereof.

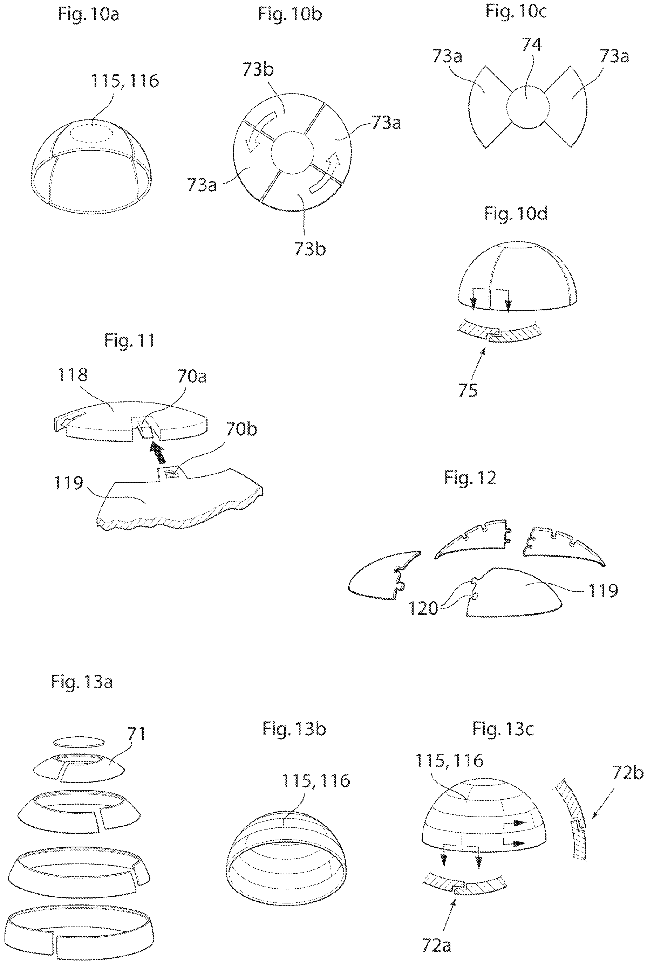

Fig. 10 shows a medical device according to an an exemplifying embodiment describing the technical area of the invention, wherein the medical device comprises a first 73a and a second 73b section, shown in fig. 10b. The first and second sections are displaceable in relation to each other. According to a first embodiment said first section 73a can be rotated in relation to said second section 73b so that said second section 73b travels underneath said first section 73a to create a displaced medical device 74, as shown in fig. 10c, which is possible to insert into a knee joint of a human patient through a hole being oval, or at least having an area smaller than the cross sectional area of the medical device when in its full functional size. According to this embodiment the two sections 73a,b are connected to each other when the medical device is returned to its full functional size using a mechanical form fitting 75, as shown in fig. 10d. However it is also conceivable that said connection is assisted or replaced with at least one screw, at least one pin, at least one portion of at least one of the parts adapted to be introduced into the other part, the parts being adapted to be sliding into the other part, form fitting, welding, adhesive, pin, wire, a ball mounted into a bowl being portions of said parts, a male portion of one part mounted into a female portion of the other part, a key introduced into a lock being portions of said parts, band, or other mechanical connecting members.

Fig. 11 shows an exemplifying embodiment describing the technical area of the invention, wherein a self locking structure comprises a male part 70a which is adapted enter into a female part 70b comprising an elastic member adapted to lock an edge of said male part 70a inside of said female part 70b in a locking position. According to one embodiment the female part 70b is attached to an artificial knee joint surface part 119 adapted to be fixated to a base part 118 for forming an assembled artificial knee joint surface part. However it is equally conceivable that the male part is attached to the knee joint surface part 119, or that neither of the interconnecting parts is a base part. The self locking members 70a,b can be assisted or replaced with at least one screw, at least one pin, at least one portion of at least one of the parts adapted to be introduced into the other part, the parts being adapted to be sliding into the other part, form fitting, welding, adhesive, pin, wire, a ball mounted into a bowl being portions of said parts, a male portion of one part mounted into a female portion of the other part, a key introduced into a lock being portions of said parts, band, or other mechanical connecting members.

Fig. 12 shows an exemplifying embodiment describing the technical area of the invention, disclosing one example of form fitted structure, wherein the artificial knee joint surface parts 119 have elements 120 similar to jigsaw puzzle pieces, which interconnects to corresponding elements of one or more artificial knee joint surface part 119.

Fig. 13a,b,c shows a medical device according to an exemplifying embodiment describing the technical area of the invention, wherein the medical device comprises multiple ring-shaped medical device parts 71. Said multiple ring-shaped medical device parts 71 are adapted to be connected to each other to form a medical device after insertion in a knee joint. According to one embodiment said medical device parts 71 are adapted to be connected to each other using mechanical connecting members 72a,b. Fig. 13c shows how an individual ring-shaped medical device part 71 can be connected to itself using the mechanical connecting member 72a to form a continuous ring shape. Further 13c shows how an individual ring-shaped medical device part 71 connects to other ring-shaped medical device parts 71 using the mechanical connecting member 72b to form a medical device.

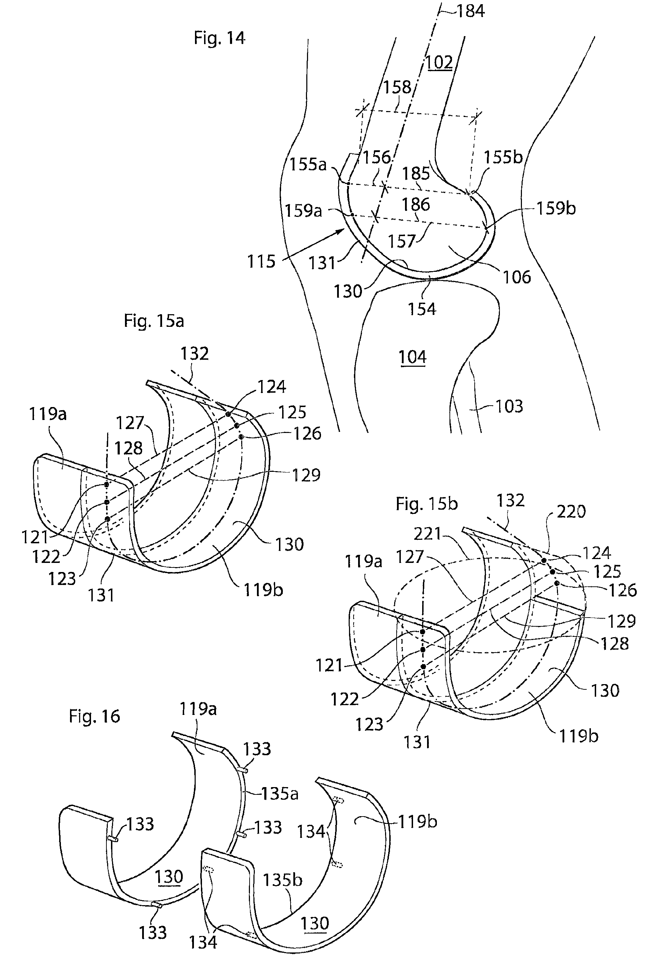

Fig. 14 shows the right leg of a human patient in section when a medical device 115 has been implanted. The medical device clasps the medial condyle and supplies a fixation by form fitting i.e. the shape of the medical device fixate the medical device to the medial condyle 106. However it is equally conceivable that the medical device is adapted to clasp the lateral condyle 105, or both the medial and the lateral condyle. The medical device 115 has a substantially horseshoe-shaped section having a base 154, two ends 155a,b, an inner surface 130 and an outer surface 131. A first straight line 156 reaches from a first 155a end of said inner surface 130 of said horseshoe-shaped section to a second end 155b of said horseshoe-shaped section, said first straight line 156 being positioned in an insertion opening 158 of said medical device 115. A second straight line 157, reaches between two points 159a,b on said inner surface 130 of said horseshoe-shaped section. The first straight 156 line and the second straight line 157 are parallel and the first straight line 156 is shorter than the second straight line 157 and the second straight 157 line is placed closer to the base 154 of the horseshoe-shaped section than the first straight line 156. According to the embodiment shown in fig 14 the second straight line is the maximum diameter of the condyle 105, 106. The medical device 115 comprises a at least a first beyond part, e.g. the part between 155b and 159b, adapted to cover and/or go into the bone of the femur 102 on at least a part of the femur 102 in the proximal direction of the elongated distribution of the femur 102. The at least one first beyond part is adapted to have a closest distance 185 to a centre axis 184 of the elongated distribution of the femur 102, being smaller than the closest distance 186 of a point of the maximum diameter 186 of the femoral condyle. Thereby a more stable position of the artificial femur surface 131 and of the medical device 115, when mounted on at least one of the femoral condyles 105, 106 in the functional position, is created.

Fig. 15 shows the medical device according to an embodiment where the medical device comprises two medical device parts 119a,b adapted to be connected after insertion in the knee joint of a human patient. According to this embodiment the medical device is adapter to have a horseshoe-shaped section and thereby being adapted to clasp the medial 106 and/or lateral 105 condyle of the femoral bone 102. The implantable medical device comprises: an inner surface 130, and an outer surface 131. The inner surface comprises: a first point 121, a second point 124, a third point 122, a fourth point 125, a fifth point 123, and a sixth point 126, all points located on different places along a length axis 132 of said inner surface 130, being a frontal to dorsal curved length axis following the curved surface. A first straight line 127, reaching from said first point 121 to said second 124 point is parallel to a second straight line 128 reaching from said third point 122 to said fourth point 125, which in turn is parallel to a third straight line 129 reaching from said fifth point 123 to said sixth point 126. The first 127 and third 129 straight lines are of equal length, and wherein said second straight line 128 is longer than said first 127 and said third 129 straight lines and positioned between said first 127 and said third 129 straight lines.

Fig. 16 shows the medical device according to an embodiment wherein the medical device comprises two medical device parts 119a,b adapted to be connected to each other after implantation in a knee joint. The first medical device part 119a comprises pins 133, as locking members, which correspond to the holes 134 in the second medical device part 119b. When the pins 133 are introduced into the holes 134 a locking position is created which is adapted substantially lock the medical device in all directions except the introduced direction and/or backwards thereof. The mechanical fixation that the construction with pins 133 and holes offer can be assisted by bone cement or an adhesive applied either to the pins 133 and holes 134, the interconnecting surfaces 135a,b of the medical device parts 119a,b, and/or to the inner surfaces 130 of the medical device being in contact with human bone. The adding of bone cement or adhesive to the fixation of the medical device parts enable the locking position to lock the medical device in the introduced direction and/or backwards thereof as well. Fig. 16 further shows an example of the medical device having a central part 1601 and a surrounding part 1602

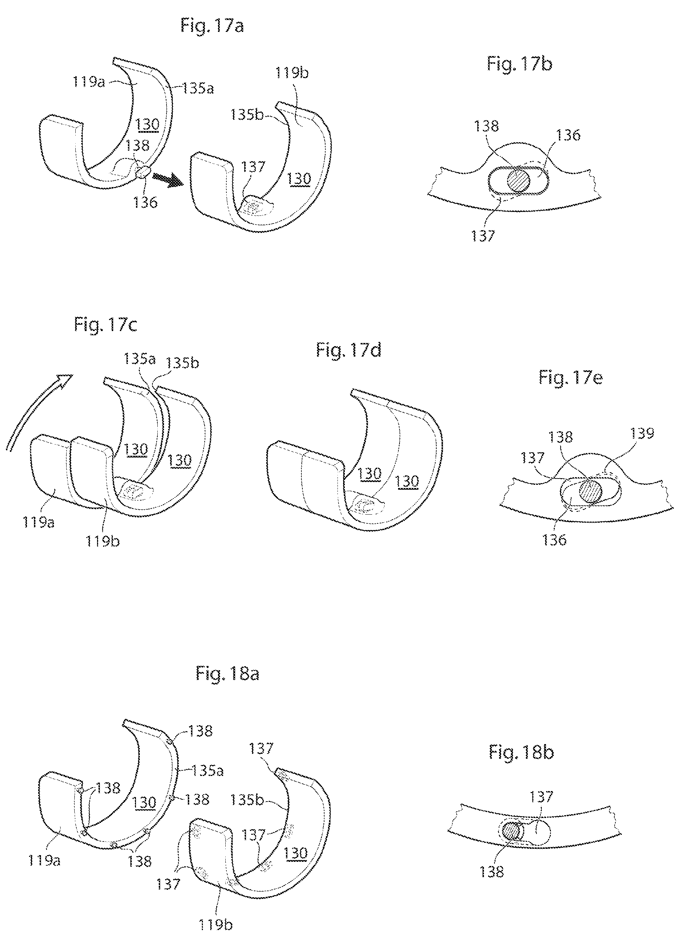

Fig. 17a shows the medical device according to an embodiment wherein the medical device parts 119a,b are mechanically fixated to each other by means of a locking member 136 rotatable around an axis 139. The locking member 138 is fixated to a medical device part 119a and corresponds with a keyhole 137 in another medical device part 119b. The locking member 138 is adapted to place the medical device in a locking position thought the form fitted structure of the locking member 138 being adapted to be introduced in at least two consecutive different directions and in the locking position. When placed in the locking position the medical device is substantially locked in all directions except the last introduced direction and/or backwards thereof.

Fig. 17b shows the locking member 138 and keyhole 137 in further detail,

Fig. 17c shows the medical device in a first state wherein the locking member is introduced to the keyhole 137 of the second medical device part 119b in a first direction.

Fig. 17d shows the medical device in a second state when the locking member 138 has been shifted an angle in the keyhole 137 fixating the locking member 138 behind the section of the keyhole 137. This fixation fixates the locking member 138 in said locking position in all directions except the last introduced direction and/or backwards thereof. The mechanical fixation that the construction with locking member 138 and keyhole 137 offer can be assisted by bone cement or an adhesive applied either to the locking member 138 and keyhole 137, the interconnecting surfaces 135a,b of the medical device parts 119a,b, and/or to the inner surfaces 130 of the medical device being in contact with human bone. The adding of bone cement or adhesive to the fixation of the medical device parts enable the locking position to lock the medical device in the last introduced direction and/or backwards thereof as well.

Fig. 17e shows the locking member 138 and keyhole 137 in further detail.

Fig. 18a shows the medical device according to another embodiment in which the interconnection of two medical device parts 119a,b is achieved through placing the two medical device parts 119a,b in a locking position using a locking member 138 and a keyhole 137. The locking member 138 is adapted to place the medical device in a locking position thought the form fitted structure of the locking member 138 being adapted to be introduced in two consecutive different directions in the locking position. When placed in the locking position the medical device is substantially locked in all directions except the last introduced direction and/or backwards thereof. The mechanical fixation that the construction with locking member 138 and keyhole 137 offer can be assisted by bone cement or an adhesive applied either to the locking member 138 and keyhole 137, the interconnecting surfaces 135a,b of the medical device parts 119a,b, and/or to the inner surfaces 130 of the medical device being in contact with human bone. The adding of bone cement or adhesive to the fixation of the medical device parts enable the locking position to lock the medical device in the last introduced direction and/or backwards thereof as well.

Fig. 18b shows the locking member 138 and keyhole 137 in further detail when the locking member 138 has been introduced into the keyhole 137 in two consecutive different directions. The locking member 138 is now placed in the locking position and the medical device is substantially locked in all directions except the last introduced direction and/or backwards thereof.

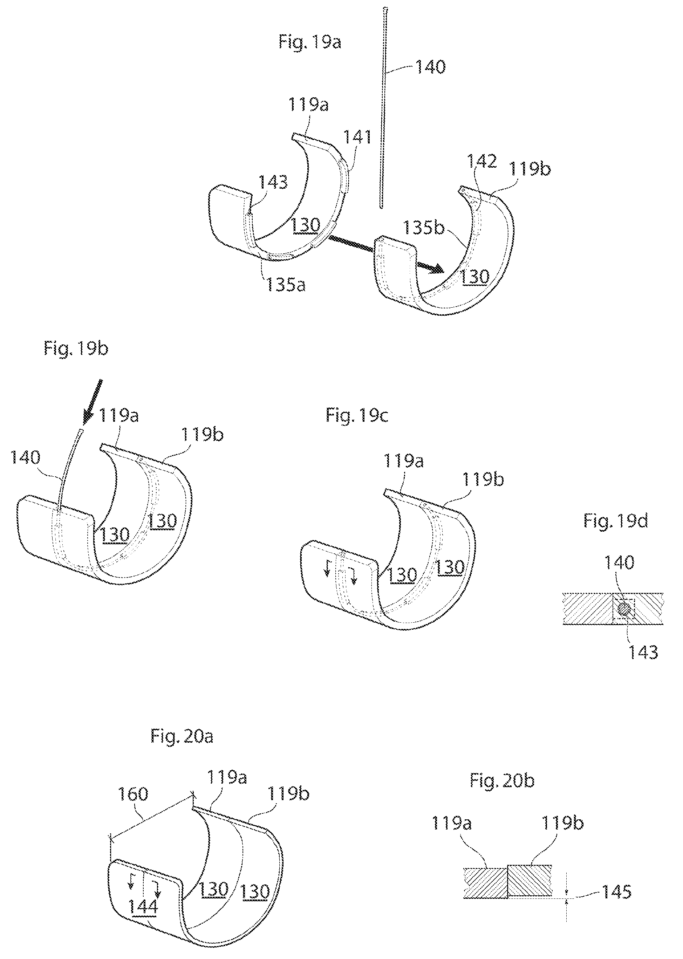

Fig. 19a shows the medical device according to an embodiment where the medical device comprises two medical device parts 119a,b adapted to be interconnected through a system of locking members 141 and holes 142. The locking members 141 of the first medical device part 119a correspond to the holes 142 of the second medical device part 119b. When the locking members 141 has been introduced to the holes 142 they are locked in their final position using a flexible member 140 adapted to be introduced into a hole 143 which runs along a length axis of the medical device and a length axis of the locking members 141.

Fig. 19b shows the medical device when the two medical device parts 119a,b has been interconnected and the locking members 141 are positioned in the holes 142 and the flexible member 140 is being introduced into a hole 143 which now runs through a length axis of the medical device.

Fig. 19c shows the medical device when the flexible member 140 is positioned in its final position.

Fig. 19d shows a cross-section of the medical device when the locking members 138 has been placed in the holes 142 and the flexible member 140 has been placed in the hole 143 which runs along a length axis of the medical device.

Fig. 20a shows the medical device according to any of the embodiments wherein two medical device parts 119a,b are interconnected to create an even surface 144 along the connection line. According to one embodiment the height difference 145 in a cross-section of said even surface is adapted to be smaller than 10 micrometer, in another embodiment the height difference 145 in a cross-section of said even surface is adapted to be smaller than 100 micrometer, and in yet another embodiment the height difference 145 in a cross-section of said even surface is adapted to be smaller than 1 millimeter. 10. Furthermore the medical device according to the embodiment shown in fig. 20a have a largest cross-sectional distance 160 which is variable by means of a movable member being an elastic member being the medical device being made of an elastic material.

Fig. 20b shows a cross-section of the medical device, indicating the height difference 145 between the medical device parts 119a,b.

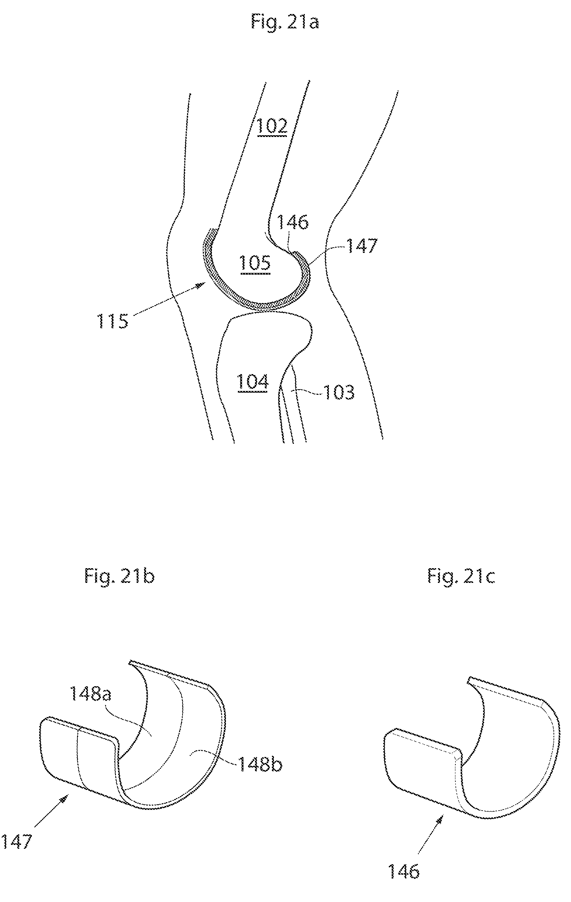

Fig. 21a shows the right leg of a human patient in section, wherein an implantable medical device 115 has been implanted. The medical device comprises an artificial surface comprising at least two layers 146,147 mounted together. The first layer 146 comprises a flexible layer 146 and the second layer comprises a carrying layer. The carrying layer comprises two carrying layer parts 148a,b which are adapted to be mounted on said flexible layer 146. Fig. 21a further shows the condyles having an imaginary complete ball shape, which abuts the periphery of the condyles.

Fig. 21b shows the two carrying layer parts 148a,b are interconnected through the mounting of the two carrying layer parts 148a,b on the flexible layer 146. The process of implanting the medical device in the knee joint of a human patient is performed by first the flexible layer 146 being placed on inside of the knee joint, and second, the first of the carrying layer parts 148a is fixated to the flexible layer 146, and third, the second carrying layer part 148b is fixated to the flexible layer 146. The first 148a and second 148b carrying layer parts are fixated to the flexible layer 146 by means of an adhesive or a mechanical fixating member such as screws. The construction with one flexible layer 146 and two carrying layer parts 148a,b enable the introduction of the medical device into the knee joint through a hole smaller than the medical device in its full functional size.

Fig. 21c shows the flexible layer 146, which could be an elastic polymer such as PE, polyurethane, silicone or an acrylic polymer.

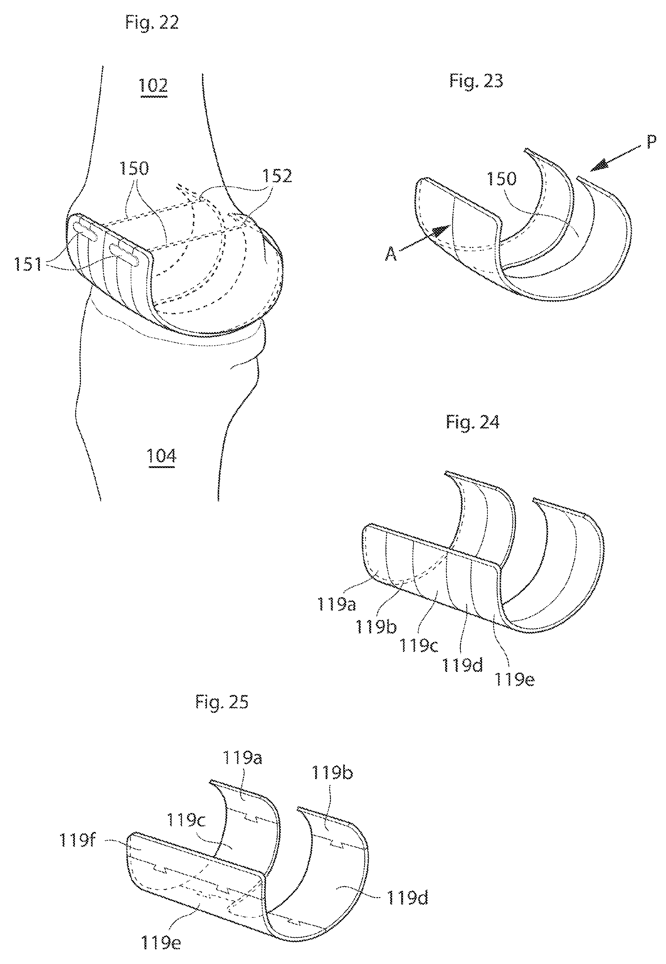

Fig. 22 shows the medical device when fixated to the medial and lateral condyle of the femoral bone of a human patient. The medical device according to this embodiment is fixated to the medial 106 and lateral 105 condyle by means of the shape of the medical device clasping the condyles 105,106 and by means of a through-going fixation element 150. The through-going fixation element 150 is adapted to travel from the frontal part of the medical device positioned at the frontal part of the femoral bone 102, through the femoral bone 102 and into the rear part of the medical device positioned on the rear part of the femoral bone 102. The through-going fixation elements 150 are attached to the medical device using a fixed arrangement 151 in one end and an adjustable arrangement 152 in the other end. A fixed arrangement could be such as a bolt head, whereas an adjustable arrangement could be such as a nut.

Fig. 23 shows the medical device according when removed from the femoral bone 102. The medical device comprises a cut-away section 150 which has been removed to make room in the medical device for the cruciate ligament placed centrally in the knee joint. According to the embodiment shown in fig. 23 the medical device is adapted to clasp both the medial and the lateral condyle, however it is equally conceivable that one of the two posterior section is adapted not the clasp the medial or lateral condyle respectively.

Fig. 24 shows the medical device according to an embodiment in which the medical device comprises five medical device parts 119a,b,c,d,e adapted to be interconnected in the operational direction of the knee joint to form the medical device. The five interconnecting medical device parts 119a,b,c,d,e could be adapted to be fixated to each other using at least one screw, at least one pin, at least one portion of at least one of the parts adapted to be introduced into the other part, the parts being adapted to be sliding into the other part, form fitting, welding, adhesive, pin, wire, a ball mounted into a bowl being portions of said parts, a male portion of one part mounted into a female portion of the other part, a key introduced into a lock being portions of said parts, band, or other mechanical connecting members.

Fig. 25 shows the medical device according to an embodiment wherein the medical device comprises five medical device parts 119a,b,c,d,e which are adapted to the interconnected in a direction perpendicular to the operational direction of the knee joint. According to the embodiment shown in fig. 25 the medical device parts 119a,b,c,d,e are interconnected by means of dovetail joints 153, however it is equally conceivable that the medical device parts 119a,b,c,d,e are interconnected using at least one screw, at least one pin, at least one portion of at least one of the parts adapted to be introduced into the other part, the parts being adapted to be sliding into the other part, form fitting, welding, adhesive, pin, wire, a ball mounted into a bowl being portions of said parts, a male portion of one part mounted into a female portion of the other part, a key introduced into a lock being portions of said parts, band, or other mechanical connecting members.

The principles of all of the above mentioned embodiments of the medical device could be adapted to serve as a joint surface fixated to the medial condyle and/or the lateral condyle, as well as the upper part of the tibia bone.

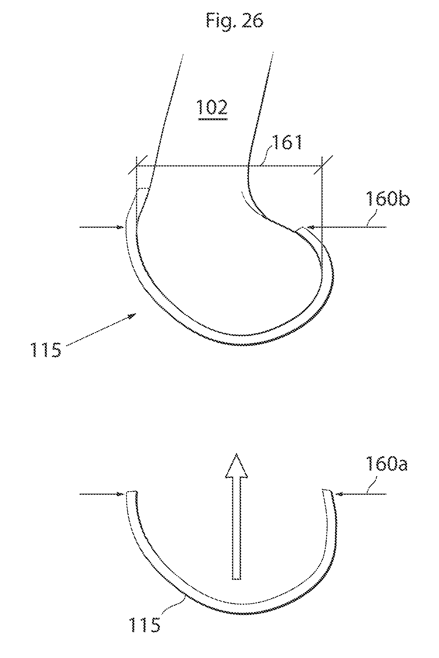

Fig. 26 shows the medical device 115 according to an embodiment wherein the distal part of the femoral bone 102 has a largest cross sectional distance 161. The medical device 115, according to this embodiment, comprises an opening 161b smaller than said largest cross sectional distance 160 of the distal portion of the femoral bone 102 when the medical device 115 is mounted on the distal part of the femoral bone 102 in a functional position. The opening 160a is larger than the largest cross sectional distance 161 of the distal part of the femoral bone 102 when the medical device 115 travels over the said distal part of the femoral bone 102.

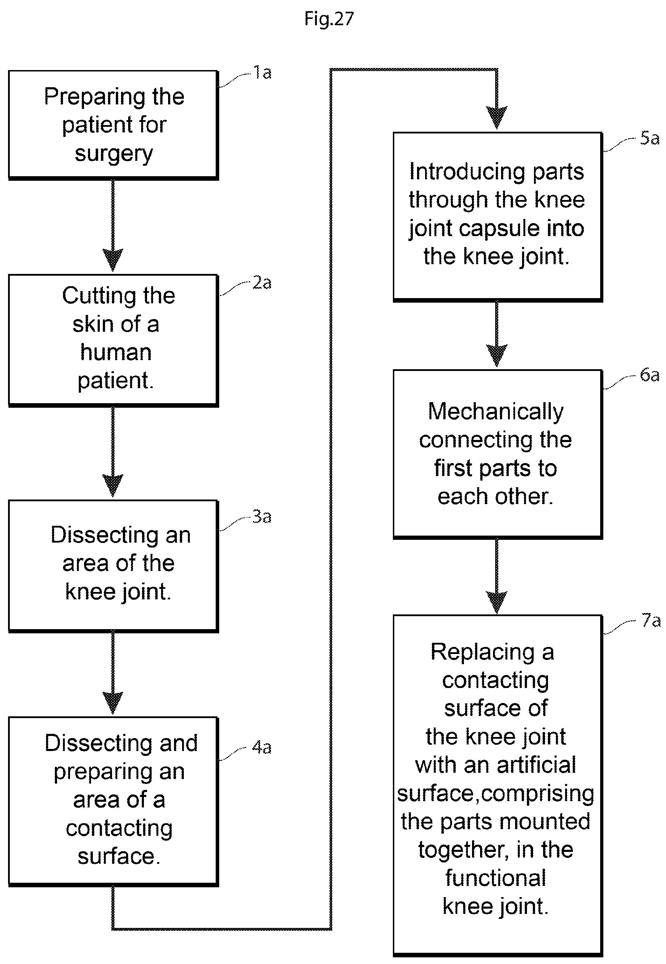

Fig. 27 shows one embodiment that includes several of the steps of a surgical method. In other embodiments, one or more steps may be omitted or performed in a different order. In step 1a, the patient is prepared for surgery in a manner that is known to a person skilled in the art. Preferably, the method is performed on the patient in the supine position. In step 2a the skin and the knee joint capsule 132 of the human patient is cut enabling the surgeon to reach the knee joint. In step 3a an area of the knee joint is dissected, after which, in step 4a, an area of a contacting surface in said knee joint is being dissected and prepared. In step 5a parts are introduced into the knee joint through the skin and the knee joint capsule. In step 6a the introduced parts are mechanically connected to each other, and in step 7a a contacting surface of the knee joint is replaced with an artificial surface, comprising the parts mounted together, in the functional knee joint. After said steps have been performed the incision is closed by suturing, taping, clamping or stapling.

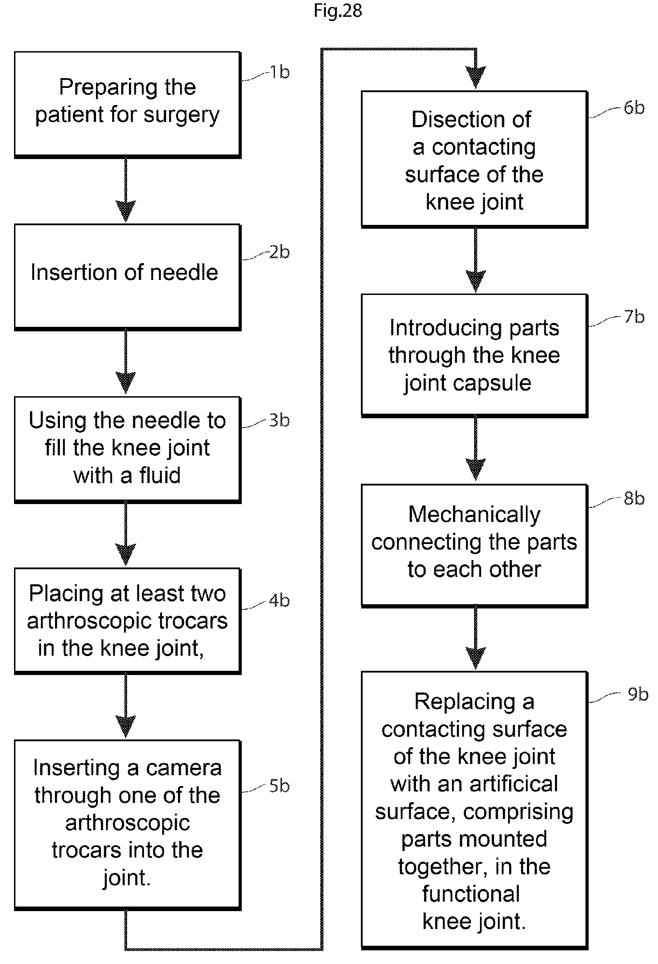

Fig. 28 shows one embodiment that includes several of the steps of an arthroscopic method. According to other embodiments, one or more step may be omitted or performed in a different order. In step 1b, the patient is prepared for surgery in a manner that is known to a person skilled in the art. Preferably, the method is performed on the patient in the supine position. In step 2b a needle is introduced into the site of operation. In step 3b, the needle is used to fill an area of the patient's knee joint with a fluid. In step 4b at least one arthroscopic trocar is insertion into the knee joint of the patient. In one embodiment at least one trocar is used for viewing the operation site and at least one trocar is used for performing various surgical step. In step 5b a camera is inserted through one of the arthroscopic trocars into the joint. In step 6b a contacting surface of the knee joint is dissected. In step 7b parts are introduced through one of the arthroscopic trocars, through the knee joint capsule and into the knee joint. In step 8b the parts are mechanically connected inside of said knee joint to form an artificial knee joint surface. In step 9b a contacting surface of the knee joint is replaced by an artificial contacting surface comprising the parts mounted together in the functional knee joint. After said steps have been performed the incision is closed by suturing, taping, clamping or stapling. According to one embodiment said fluid is circled from an inlet to said knee joint to an outlet from said knee joint, for keeping said fluid transparent.

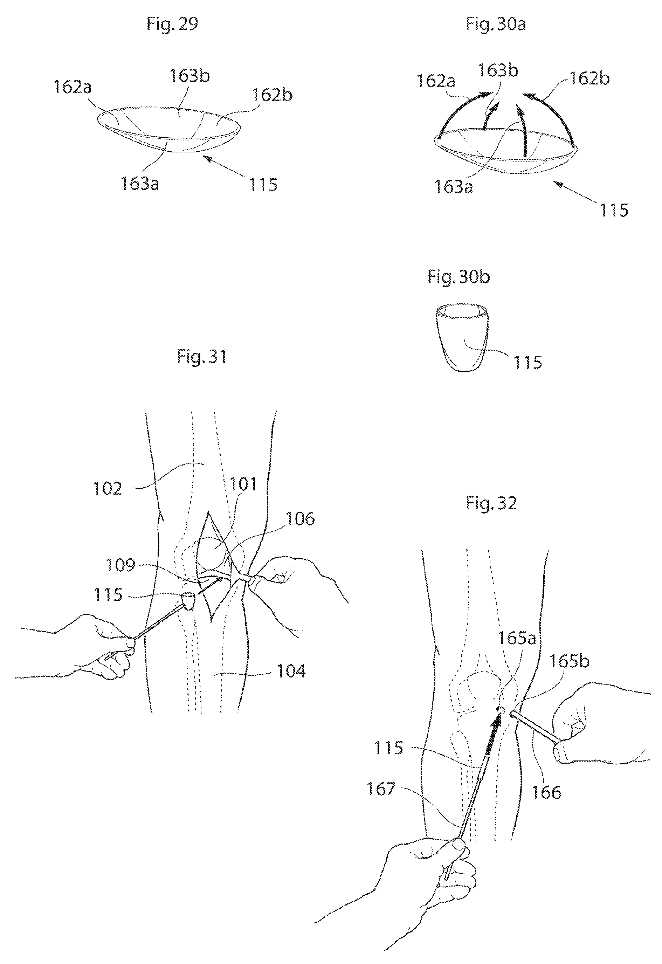

Fig. 29 shows an an exemplifying embodiment describing the technical area of the invention, wherein a medical device 115 comprises four moveable medical device sections 162a,b, 163a,b connected to each other. The four moveable medical device sections 162a,b, 163a,b are moveable by means of the medical device 115 being made of an elastic material.

Fig. 30a shows the medical device according to the embodiment of fig. 29 in a first state. The two moveable medical device sections 162a,b are adapted to be folded towards the centre of the medical device 115 before the other two moveable medical device sections 163a,b.

Fig. 30b shows the medical device 115 according to the embodiment of fig. 29 in a second state wherein all four moveable medical device sections 162a,b 163a,b have been folded towards the centre of the medical device 115. This state enables the insertion of the medical device 115 into the knee joint of a human patient through a hole smaller than the medical device 115 in its full functional size.

Fig. 31 shows right leg of a human patient when the medical device 115 according to the embodiment in fig. 29 and 30 is being inserted into the knee joint of a human patient in a surgical method.

Fig. 32 shows the right leg of a human patient when the medical device 115 according to the embodiment in fig. 29 and 30 is being inserted into the knee joint of a human patient in a arthroscopic method. The medical device is inserted through a first trocar 165a using a medical device inserter 167. Furthermore the surgeon uses a second trocar 165b for placing a camera 166 inside of the knee joint for viewing the steps of the surgery.

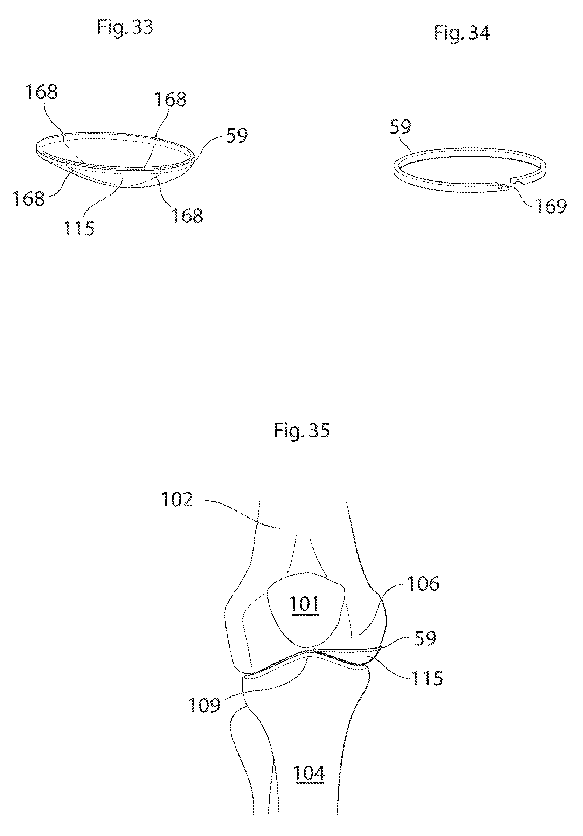

Fig. 33 shows an exemplifying embodiment describing the technical area of the invention, wherein a medical device comprises four slits 168 adapted to make the medical device elastic for clasping the medial 106 or lateral 105 condyle, or a cross-section of the medial 106 or lateral 105 condyle. The medical device is the fixated to the medial 106 or lateral 105 condyle, or a cross-section of the medial 106 or lateral 105 condyle using a band 59 placed in a cut on the upper part of the medical device 115.

Fig. 34 shows the band adapted to fixate the medical device according to the embodiment of fig. 33 to the medial 106 or lateral 105 condyle, or a cross-section of the medial 106 or lateral 105 condyle. The band 159 is secured by means of a self locking member 169.

Fig. 35 shows the medical device according to the embodiment of fig. 33 and 34 when the medical device is fixated to a cross-section of the medial condyle 106 of the femoral bone 102 using the band 59.

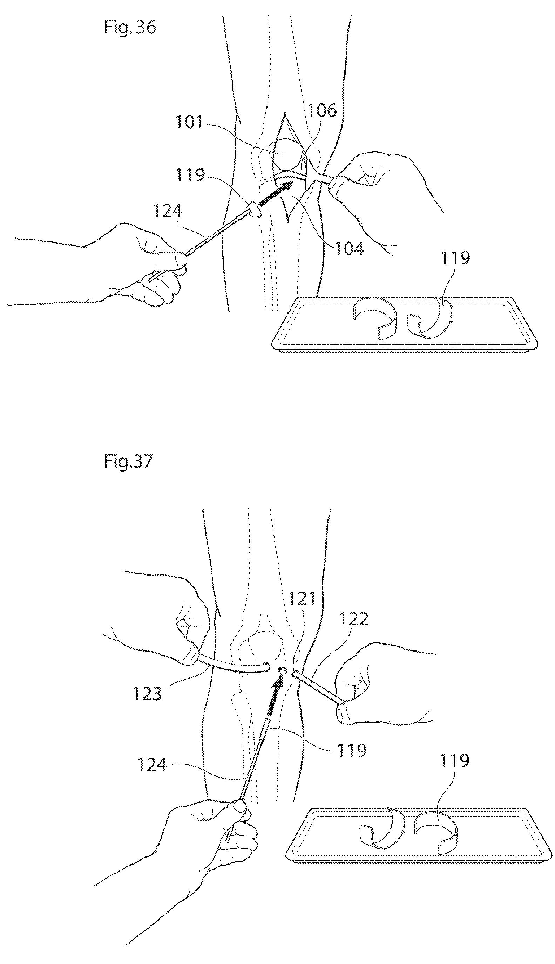

Fig. 36 shows a frontal view of the right leg of a human patient when a surgical method is being performed. Artificial knee joint surface parts 119 are inserted through the skin and the knee joint capsule 132 and into the knee joint. After the artificial knee joint surface parts have been inserted through the knee joint capsule 132 they are mechanically connected and fixated to a carrying surface of said knee joint, which could be a surface of the tibia bone 104, the medial condyle 106 of the femoral bone 102 or the lateral condyle 105 of the femoral bone 102, or any combination thereof.

Fig. 37 shows a frontal view of the right leg of a human patient when an arthroscopic method is performed. A surgical instrument 124 for inserting the parts 119 through an arthroscopic trocar 121, a viewing instrument 123 for viewing the inside of the knee joint through an and an arthroscopic trocar 121 and an instrument adapted to circulate a fluid inside of said knee joint for keeping said fluid transparent is provided. After the insertion of said artificial knee joint surface parts has been concluded the instrument for inserting the parts 119 through an arthroscopic trocar 121 is preferably replaced by an instrument adapted to mechanically connect at least one artificial knee joint surface parts into a mounted artificial knee joint surface.

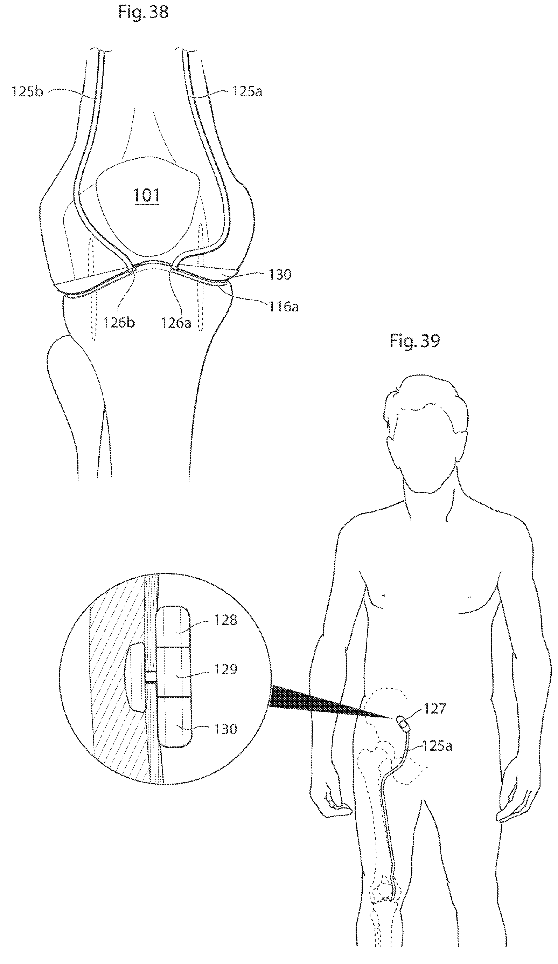

Fig. 38 shows an implantable lubricating system adapted to lubricate the artificial knee joint surfaces after they have been implanted in said knee joint. According to the embodiment shown the lubrication system comprises two lubricating fluid transport members 125a, 125b attached to two lubricating fluid injecting members 126a, 126b located at an area of the knee joint. The first lubricating fluid transport member 125a is adapted to lubricate the artificial knee joint surface 130 placed on the medial condyle 106 and the artificial knee joint surface 116a on the medial side of the tibia bone 104. The second lubricating fluid transport member 125b is adapted to lubricate the artificial knee joint surface placed on the lateral condyle 105 and the artificial knee joint surface on the lateral side of the tibia bone 104. The lubricating fluid in intermittently or continuously introduced into the joint and the lubricating fluid is preferably a biocompatible lubricating fluid such as hyaluronic acid.
Fig. 39 shows the implantable lubricating system when a central unit 127 is subcutaneously placed in the abdominal region of the patient and the lubricating fluid transport members 125a, 125b is attached to the central unit 127. The central unit preferably comprises a pumping member 130 adapted to pump the lubricating fluid, a container 129 adapted to hold said lubricating fluid and an injection port 128 for filling said container 129. It is furthermore conceivable that the central unit comprises control logic for controlling the inflow of lubricating fluid into said knee joint.

Please note that any embodiment or part of embodiment could be combined in any way. All examples herein should be seen as part of the general description and therefore possible to combine in any way in general terms.

## Claims

1. A medical device (115) for implantation in a knee joint of a human patient, said medical device having an outer surface (131), being curved, creating a curved outer surface along a frontal to dorsal curved length axis (132) following the curved outer surface, having a middle section, when said medical device is implanted in a functional position in the knee joint, said middle section being placed in the middle of said curved outer surface along said length axis, said medical device comprising:
- a form fitted structure clasping the distal portion of the femoral bone,
- at least one beyond part, extending from the surrounding part of the curved outer surface beyond an equatorial plane of an equatorial half of the condyle, extending away from the contacting surface, on at least one condyle, on at least a part of the curved outer surface, wherein the at least one first beyond part is adapted to have a closest distance (185) to a centre axis (184) of the elongated distribution of the femur (102), being smaller than the closest distance (186) of a point of the maximum diameter of the femoral condyle, the beyond part further being adapted to create a more stable position of the medical device, when mounted on at least one of the femoral condyles, when implanted in a functional position in the knee joint, and
- a through-going fixation element (150) in the at least one beyond part, adapted to travel from the frontal part of said medical device positioned at the frontal part of the femoral bone, through the femoral bone and into the rear part of the medical device positioned on the rear part of the femoral bone, to fixate the medical device clasping the distal portion of the femoral bone by the through-going fixation element, when mounted in the knee joint, **characterized in that**
- at least two parts (119a, 119b) are adapted to be connected to each other after implantation in the knee joint of the human patient, wherein the two parts is mounted with a form fitted structure (137, 138) along a length axis of the medical device along the functional movement of the knee joint.

2. The medical device according to claim 1, wherein the medical device comprises a first articulating surface adapted replaced the surface of the contacting surface of the medial condyle, a second articulating surface adapted to replace the contacting surface of the lateral condyle and a third articulating surface adapted to replace the contacting surface of the patella.

3. The medical device according to any one of claims 1-2, wherein said through-going fixation element is attached to the medical device using a fixed arrangement at one portion and an adjustable arrangement at the other portion of the medical device.

4. The medical device according to any one of claims 1-3, wherein through-going fixation element comprises at least one of,
a threaded portion and wherein the adjustable arrangement comprises a portion with corresponding threads,
screws,
pins,
wire,
rivets,
band, and
cord.

5. The medical device according to any one of the preceding claims, wherein the medical device is adapted to be placed on an intact distal portion of the femoral bone or at least to an overwhelming part towards cortical femoral bone.

6. The medical device according to any one of claims 1-5, wherein said medical device has a substantially horseshoe-shaped section having an inner surface and an outer surface, wherein:
a. a first straight line, reaches from a first end of said inner surface of said horseshoe-shaped section to a second end of said horseshoe-shaped section, said first straight line being positioned in an insertion opening of said medical device,
b. a second straight line, reaches between two points on said inner surface of said horseshoe-shaped section, wherein:
i. said first straight line and said second straight line are parallel, and
ii. said first straight line is shorter than said second straight line.

7. The medical device according to any one of claims 1-6, wherein the medical device further comprises a material adapted to be positioned between the medical device and a contacting surface of the femoral bone.

8. The medical device according to claim 7, wherein said material is a material selected from a group consisting of: adhesive materials, elastic materials and bone cement.

9. The medical device according to any one of claims 1-6, wherein the medical device further comprises a material adapted to be positioned between the medical device and a contacting surface of the tibia bone or an artificial replacement therefore, wherein said material is a material adapted to reduce friction in the knee joint.

10. The medical device according to claim 9, wherein said material adapted to reduce friction is at least one of; a lubricating fluid, and a fluorpolymer material.

11. The medical device according to anyone of the preceding claims, wherein the medical device is at least one of; adapted to be fixated to a bone of the human patient using at least one fixation element, and comprising at least two parts adapted to be connected to each other after implantation in the knee joint of the human patient using at least one element, wherein
said element is selected from a group consisting of:
- at least one screw,
- at least one pin,
- at least one portion of at least one of the parts adapted to be introduced into the other part,
- the parts being adapted to be sliding into the other part,
- form fitting,
- welding,
- adhesive,
- pin,
- wire,
- a ball mounted into a bowl being portions of said parts,
- a male portion of one part mounted into a female portion of the other part,
- a key introduced into a lock being portions of said parts,
- band, and
- other mechanical connecting members.

12. The medical device according to any one of the preceding claims, wherein the medical device is adapted to be fixated to the distal part of the femoral bone without penetration of the cortex of the femoral bone.

13. The medical device according to any one of the preceding claims, wherein said medical device comprises a biocompatible metal material.

14. The medical device according to claim 13, wherein said biocompatible metal material is at least one of; titanium and tantalum.

15. The medical device according to any one of the preceding claims, wherein the medical device, comprise at least two layers mounted together.

## Patentansprüche

1. Medizinische Vorrichtung (115) zur Implantation in ein Kniegelenk eines menschlichen Patienten, wobei die medizinische Vorrichtung eine Außenfläche (131) aufweist, die geborgen ist, wodurch eine gebogene Außenfläche entlang einer von frontal zu dorsal gebogenen Längsachse (132) erzeugt wird, die der gebogenen Außenfläche folgt, und einen mittleren Abschnitt aufweist, wenn die medizinische Vorrichtung in einer Funktionsstellung im Kniegelenk implantiert ist, wobei der mittlere Abschnitt in der Mitte der gebogenen Außenfläche entlang der Längsachse platziert ist, wobei die medizinische Vorrichtung Folgendes umfasst:
- eine eng anliegende Struktur, die den distalen Abschnitt des Oberschenkelknochens umklammert,
- wenigstens einen sich darüber hinaus erstreckenden Teil, der sich von dem umgebenden Teil der gebogenen Außenfläche über eine Äquatorialebene einer Aquatorialhälfte des Kondylus hinaus erstreckt, sich von der Kontaktfläche weg erstreckt, auf wenigstens einem Kondylus, auf wenigstens einem Teil der gebogenen Außenfläche, wobei der wenigstens eine sich darüber hinaus erstreckende Teil ausgelegt ist, um einen engsten Abstand (185) zu einer Mittelachse (184) der Längsverteilung des Oberschenkelknochens (102) aufzuweisen, der kleiner als der engste Abstand (186) eines Punkts des maximalen Durchmessers des Femurkondylus ist, wobei der sich darüber hinaus erstreckende Teil ferner ausgelegt ist, um eine stabilere Position der medizinischen Vorrichtung zu erzeugen, wenn sie an wenigstens einem der Femurkondylen angebracht ist, bei Implantation in einer Funktionsstellung in dem Kniegelenk, und
- ein durchgehendes Fixierungselement (150) in dem wenigstens einen sich darüber hinaus erstreckenden Teil, das ausgelegt ist, um sich von dem frontalen Teil der medizinischen Vorrichtung am frontalen Teil des Oberschenkelknochens durch den Oberschenkelknochen und in den hinteren Teil der medizinischen Vorrichtung auf dem hinteren Teil des Oberschenkelknochens zu bewegen, um die medizinische Vorrichtung zu fixieren, wobei der distale Abschnitt des Oberschenkelknochens von dem durchgehenden Fixierungselement umklammert wird, wenn es in dem Kniegelenk angebracht ist, **dadurch gekennzeichnet, dass**
- wenigstens zwei Teile (119a, 119b) ausgelegt sind, um nach Implantation in dem Kniegelenk des menschlichen Patienten miteinander verbunden zu werden, wobei die beiden Teile mit einer eng anliegenden Struktur (137, 138) entlang einer Längsachse der medizinischen Vorrichtung entlang der Funktionsbewegung des Kniegelenks angebracht sind.

2. Medizinische Vorrichtung nach Anspruch 1, wobei die medizinische Vorrichtung eine erste Gelenkfläche, die ausgelegt ist, um die Oberfläche der Kontaktfläche des medialen Kondylus zu ersetzen, eine zweite Gelenkfläche, die ausgelegt ist, um die Kontaktfläche des lateralen Kondylus zu ersetzen, und eine dritte Gelenkfläche, die ausgelegt ist, um die Kontaktfläche der Kniescheibe zu ersetzen, umfasst.

3. Medizinische Vorrichtung nach einem der Ansprüche 1-2, wobei das durchgehende Fixierungselement unter Verwendung einer fixierten Anordnung an einem Abschnitt und einer verstellbaren Anordnung an dem anderen Abschnitt der medizinischen Vorrichtung an der medizinischen Vorrichtung befestigt ist.

4. Medizinische Vorrichtung nach einem der Ansprüche 1-3, wobei das durchgehende Fixierungselement wenigstens eines der Folgenden umfasst:
einen Gewindeabschnitt, und wobei die verstellbare Anordnung einen Abschnitt mit entsprechenden Gewinden umfasst,
Schrauben,
Stifte,
Draht,
Nieten,
Band, und
Schnur.

5. Medizinische Vorrichtung nach einem der vorhergehenden Ansprüche, wobei die medizinische Vorrichtung ausgelegt ist, auf einem intakten distalen Abschnitt des Oberschenkelknochens oder wenigstens bis zu einem überwiegenden Teil zum kortikalen Oberschenkelknochen platziert zu werden.

6. Medizinische Vorrichtung nach einem der Ansprüche 1-5, wobei die medizinische Vorrichtung einen im Wesentlichen hufeisenförmigen Abschnitt mit einer Innenfläche und einer Außenfläche aufweist, wobei:
a. eine erste Gerade von einem ersten Ende der Innenfläche des hufeisenförmigen Abschnitts bis zu einem zweiten Ende des hufeisenförmigen Abschnitts reicht, wobei die Gerade in einer Einführöffnung der medizinischen Vorrichtung angeordnet ist,
b. eine zweite Gerade zwischen zwei Punkten auf der Innenfläche des hufeisenförmigen Abschnitts verläuft, wobei:
i. die erste Gerade und die zweite Gerade parallel sind, und
ii. die erste Gerade kürzer als die zweite Gerade ist.

7. Medizinische Vorrichtung nach einem der Ansprüche 1-6, wobei die medizinische Vorrichtung ferner ein Material umfasst, das ausgelegt ist, um zwischen der medizinischen Vorrichtung und einer Kontaktfläche des Oberschenkelknochens platziert zu werden.

8. Medizinische Vorrichtung nach Anspruch 7, wobei das Material ein Material ist, das aus einer aus Klebematerialien, elastischen Materialien und Knochenzement bestehenden Gruppe ausgewählt ist.

9. Medizinische Vorrichtung nach einem der Ansprüche 1-6, wobei die medizinische Vorrichtung ferner ein Material umfasst, das ausgelegt ist, um zwischen der medizinischen Vorrichtung und einer Kontaktfläche des Tibiaknochens oder eines künstlichen Ersatzes dafür platziert zu werden, wobei das Material ausgelegt ist, um Reibung im Kniegelenk zu reduzieren.

10. Medizinische Vorrichtung nach Anspruch 9, wobei das Material, das zur Reduzierung von Reibung ausgelegt ist, wenigstens eines der Folgenden ist: eine Schmierflüssigkeit und ein Fluorpolymer-Material.

11. Medizinische Vorrichtung nach einem der vorhergehenden Ansprüche, wobei die medizinische Vorrichtung wenigstens eine der Folgenden ist:
ausgelegt, um an einem Knochen des menschlichen Patienten unter Verwendung wenigstens eines Fixierungselements befestigt zu werden, und umfassend wenigstens zwei Teile, die ausgelegt sind, um nach Implantation im Kniegelenk des menschlichen Patienten unter Verwendung wenigstens eines Elements miteinander verbunden zu werden, wobei
das Element aus der aus Folgenden bestehenden Gruppe ausgewählt ist:
- wenigstens eine Schraube,
- wenigstens ein Stift,
- wenigstens ein Abschnitt wenigstens eines der Teile, der ausgelegt ist, um in den anderen Teil eingeführt zu werden,
- wobei die Teile ausgelegt sind, um in den anderen Teil zu gleiten,
- eng anliegend,
- Schweißverbindung,
- Klebstoff,
- Stift,
- Draht,
- in einer Pfanne befestigte Kugel als Abschnitte der Teile,
- ein Steckabschnitt eines Teils, der in einem Aufnahmeabschnitt des anderen Teils angebracht ist,
- ein in ein Schloss eingeführter Schlüssel als Abschnitte der Teile,
- Band, und
- andere mechanische Verbindungselemente.

12. Medizinische Vorrichtung nach einem der vorhergehenden Ansprüche, wobei die medizinische Vorrichtung ausgelegt ist, um an dem distalen Teil des Oberschenkelknochens ohne Penetration der Kortex des Oberschenkelknochens fixiert zu werden.

13. Medizinische Vorrichtung nach einem der vorhergehenden Ansprüche, wobei die medizinische Vorrichtung ein biokompatibles Metallmaterial umfasst.

14. Medizinische Vorrichtung nach Anspruch 13, wobei das biokompatible Metallmaterial wenigstens eines der Folgenden ist: Titan und Tantal.

15. Medizinische Vorrichtung nach einem der vorhergehenden Ansprüche, wobei die medizinische Vorrichtung wenigstens zwei aneinander befestigte Schichten umfasst.

## Revendications

1. Dispositif médical (115) à implanter dans une articulation du genou d'un patient humain, ledit dispositif médical comportant une surface extérieure (131), étant courbe, créant une surface extérieure courbe le long d'un axe longitudinal (132) courbe d'avant en arrière suivant la surface extérieure courbe, ayant une section médiane, quand ledit dispositif médical est implanté dans une position fonctionnelle dans l'articulation du genou, ladite section médiane étant placée au milieu de ladite surface extérieure courbe le long dudit axe longitudinal, ledit dispositif médical comprenant :
une structure en liaison de forme serrant la partie distale du fémur,
au moins une pièce au-delà, s'étendant depuis la partie périphérique de la surface extérieure courbe au-delà du plan équatorial d'une moitié équatoriale du condyle, s'éloignant de la surface de contact, sur au moins un condyle, sur au moins une partie de la surface extérieure courbe, dans lequel ladite première pièce au-delà est apte à avoir une distance (185) la plus courte à un axe central (184) de la répartition allongée du fémur (102), qui est inférieure à la distance (186) la plus courte d'un point du diamètre maximal du condyle fémoral, la pièce au-delà étant en outre apte à créer une position plus stable du dispositif médical, quand il est monté sur au moins l'un des condyles fémoraux, lorsqu'il est implanté dans une position fonctionnelle dans l'articulation du genou, et
un élément de fixation (150) traversant dans ladite pièce au-delà, apte à se déplacer depuis la partie frontale dudit dispositif médical placé à la partie frontale du fémur, traversant le fémur et pénétrant dans la partie arrière du dispositif médical placé sur la partie arrière du fémur, pour fixer le dispositif médical serrant la partie distale du fémur à l'aide de l'élément de fixation traversant, quand il est monté dans l'articulation du genou,
**caractérisé en ce qu'**au moins deux pièces (119a, 119b) sont aptes à être raccordées l'une à l'autre après implantation dans l'articulation du genou du patient humain, dans lequel les deux pièces sont montées au moyen d'une structure (137, 138) en liaison de forme disposée suivant l'axe longitudinal du dispositif médical suivant le mouvement fonctionnel de l'articulation du genou.

2. Dispositif médical selon la revendication 1, comprenant une première surface d'articulation apte à remplacer la surface de la surface de contact du condyle interne, une deuxième surface d'articulation apte à remplacer la surface de contact du condyle externe et une troisième surface d'articulation apte à remplacer la surface de contact de la patella.

3. Dispositif médical selon la revendication 1 ou 2, dans lequel ledit élément de fixation traversant est fixé au dispositif médical au moyen d'un agencement fixe au niveau d'une partie et d'un agencement réglable au niveau d'une autre partie du dispositif médical.

4. Dispositif médical selon l'une quelconque des revendications 1 à 3, dans lequel l'élément de fixation traversant comprend au moins une partie filetée et dans lequel l'agencement réglable comprend une partie ayant un filetage homologue, vis, broches, fil, rivets, bande et cordon.

5. Dispositif médical selon l'une quelconque des revendications précédentes, dans lequel le dispositif médical est apte à être placé sur une partie distale intacte du fémur ou au moins dans une proportion écrasante vers le fémur cortical.

6. Dispositif médical selon l'une quelconque des revendications 1 à 5, dans lequel ledit dispositif médical présente une section sensiblement en forme de fer à cheval comportant une surface intérieure et une surface extérieure, dans laquelle :
a. une première ligne droite court d'une première extrémité de ladite surface intérieure de ladite section en forme de fer à cheval à une seconde extrémité de ladite section en forme de fer à cheval, ladite première ligne droite étant placée dans une ouverture d'insertion dudit dispositif médical,
b. une seconde ligne droite court entre deux points sur ladite surface intérieure de ladite section en forme de fer à cheval, dans laquelle :
i. ladite première ligne droite et ladite seconde ligne droite sont parallèles, et
ii. ladite première ligne droite est plus courte que ladite seconde ligne droite.

7. Dispositif médical selon l'une quelconque des revendications 1 à 6, comprenant en outre un matériau apte à être placé entre le dispositif médical et une surface de contact du fémur.

8. Dispositif médical selon la revendication 7, dans lequel ledit matériau est un matériau choisi dans le groupe constitué de matières adhésives, matières élastiques et ciment orthopédique.

9. Dispositif médical selon l'une quelconque des revendications 1 à 6, comprenant en outre un matériau apte à être placé entre le dispositif médical et une surface de contact du tibia ou un remplacement artificiel de celle-ci, dans lequel ledit matériau est un matériau apte à réduire le frottement dans l'articulation du genou.

10. Dispositif médical selon la revendication 9, dans lequel ledit matériau apte à réduire le frottement est un fluide de lubrification et/ou un polymère fluoré.

11. Dispositif médical selon l'une quelconque des revendications précédentes, dans lequel le dispositif médical est apte à être fixé à un os du patient humain au moyen d'au moins un élément de fixation et/ou comprend au moins deux parties aptes à être raccordées l'une à l'autre après implantation dans l'articulation du genou du patient humain au moyen d'au moins un élément, dans lequel ledit élément est choisi dans le groupe constitué de :
- au moins une vis,
- au moins une broche,
- au moins une partie d'au moins une des pièces apte à être introduite dans l'autre pièce,
- les pièces étant aptes à coulisser dans l'autre pièce,
- liaison de forme,
- soudage,
- adhésif,
- broche,
- fil,
- une boule montée dans un bol étant des parties desdites pièces,
- une partie mâle d'une pièce étant montée dans une partie femelle de l'autre pièce,
- une clé introduite dans un verrou étant des parties desdites pièces,
- bande, et
- autres éléments mécaniques de liaison.

12. Dispositif médical selon l'une quelconque des revendications précédentes, dans lequel le dispositif médical est apte à être fixé à la partie distale du fémur sans pénétrer dans la zone corticale du fémur.

13. Dispositif médical selon l'une quelconque des revendications précédentes, comprenant un matériau métallique biocompatible.

14. Dispositif médical selon la revendication 13, dans lequel ledit matériau métallique biocompatible est le titane et/ou le tantale.

15. Dispositif médical selon l'une quelconque des revendications précédentes, comprenant au moins deux couches assemblées.
